# EUROPEAN PATENT SPECIFICATION

(11) **EP 2 076 496 B1**
(45) Date of publication and mention of the grant of the patent: **31.03.2010**
(21) Application number: 07821037.4
(22) Date of filing: 09.10.2007
(51) Int. Cl.: C07D 233/64, C07D 401/12, C07D 403/12, C07D 409/12, A61K 31/417, A61P 25/00

(54) **AMINOMETHYL-2-IMIDAZOLES WITH AFFINITY WITH THE TRACE AMINE ASSOCIATED RECEPTORS**
AMINOMETHYL-2-IMIDAZOLE MIT AFFINITÄT ZU MIT DEM TRACE-AMIN ASSOZIIERTEN REZEPTOREN
AMINOMÉTHYL- 2 -IMIDAZOLES À AFFINITÉ AVEC LES RÉCEPTEURS ASSOCIÉS À UNE AMINE À L'ÉTAT DE TRACE

(30) Priority: 19.10.2006 EP 06122553
(43) Date of publication of application: 08.07.2009
(73) Proprietor: F. Hoffmann-La Roche AG, 4070 Basel (CH)
(72) Inventor: GALLEY, Guido, 79618 Rheinfelden (DE); GOERGLER, Annick, 68000 Colmar (FR); GROEBKE ZBINDEN, Katrin, 4410 Liestal (CH); NORCROSS, Roger, 4305 Olsberg (CH); STALDER, Henri, 4055 Basel (CH)
(74) Representative: Poppe, Regina
(86) International application number: PCT/EP2007/060664
(87) International publication number: WO 2008/046756

(56) References cited:
- WO-A-98/12183
- WO-A-2004/014898
- WO-A-2006/119411
- LEE SUNKYUNG ET AL: "4-[(N-imidazol-2-ylmethyl)anilino]pyranop yridine analogs as novel anti-angiogenic agents" BULLETIN OF THE KOREAN CHEMICAL SOCIETY, KOREAN CHEMICAL SOCIETY, SEOUL, KR, vol. 26, no. 4, 20 April 2005 (2005-04-20), pages 619-628, XP002429858 ISSN: 0253-2964
- LINDEMANN LOTHAR ET AL: "Trace amine-associated receptors form structurally and functionally distinct subfamilies of novel G protein-coupled receptors" GENOMICS, ACADEMIC PRESS, SAN DIEGO, US, vol. 85, no. 3, March 2005 (2005-03), pages 372-385, XP002357177 ISSN: 0888-7543

## Description

The present invention relates to compounds of formula I 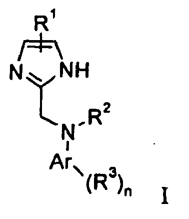 wherein
- R¹: is hydrogen or lower alkyl;
- R²: is lower alkyl, lower alkenyl, lower alkyl substituted by hydroxy,
lower alkyl substituted by halogen,
-(CH₂)ₓ-S-lower alkyl,
-(CH₂)ₓ-O-lower alkyl,
-(CH₂)ₓ-NHC(O)O-lower alkyl,
-(CH₂)ₓ-aryl or
- (CH₂)ₓ-heteroaryl;
- R³: is hydrogen, lower alkyl, lower alkoxy, halogen, hydroxy, lower alkyl substituted by halogen,
-O-(CH₂)ₘ-aryl,
-O-(CH₂)ₘ-heteroaryl,
-(CR₂)ₘ-aryl or
-(CR₂)ₘ-heteroaryl;
- R: is hydrogen, lower alkyl or hydroxy;
- Ar: is phenyl, pyrimidin-2-yl, pyrimidin-4-yl or pyridin-3-yl;
- n: is 0, 1 or 2; when n is 2, then the two R³ groups may or may not be the same;
- x: is 0, 1, 2 or 3;
- m: is 0 or 1; when m is 1, then the two R groups may or may not be the same;
and to their pharmaceutically active salts, with the exception of the following known compounds:
benzyl-(*1H*-imidazol-2-ylmethyl)-phenyl-amine (CAS 371974-08-2) and ethyl-(*1H*-imidazol-2-ylmethyl)-phenyl-amine (CAS 35294-97-4).

The specific compounds excluded from the scope of new compounds of formula I are described for example in the below mentioned references or are enclosed in public chemical libraries.
The invention includes all racemic mixtures, all their corresponding enantiomers and/or optical isomers.
In addition, all tautomeric forms of compounds of formula I are also encompassed by the present invention.

It has been found that the compounds of formula I have a good affinity to the trace amine associated receptors (TAARs), especially for TAAR1. The compounds may be used for the treatment of depression, anxiety disorders, bipolar disorder, attention deficit hyperactivity disorder (ADHD), stress-related disorders, psychotic disorders such as schizophrenia, neurological diseases such as Parkinson's disease, neurodegenerative disorders such as Alzheimer's disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.
The classical biogenic amines (serotonin, norepinephrine, epinephrine, dopamine, histamine) play important roles as neurotransmitters in the central and peripheral nervous system [1]. Their synthesis and storage, as well as their degradation and reuptake after release are tightly regulated. An imbalance in the levels of biogenic amines is known to be responsible for the altered brain function under many pathological conditions [2-5]. A second class of endogenous amine compounds, the so-called trace amines (TAs) significantly overlap with the classical biogenic amines regarding structure, metabolism and subcellular localization. The TAs include p-tyramine, β-phenylethylamine, tryptamine and octopamine, and they are present in the mammalian nervous system at generally lower levels than classical biogenic amines [6].
Their dysregulation has been linked to various psychiatric diseases like schizophrenia and depression [7] and for other conditions like attention deficit hyperactivity disorder, migraine headache, Parkinson's disease, substance abuse and eating disorders [8,9].
For a long time, TA-specific receptors had only been hypothesized based on anatomically discrete high-affinity TA binding sites in the CNS of humans and other mammals [10,11]. Accordingly, the pharmacological effects of TAs were believed to be mediated through the well known machinery of classical biogenic amines, by either triggering their release, inhibiting their reuptake or by "crossreacting" with their receptor systems [9,12,13]. This view changed significantly with the recent identification of several members of a novel family of GPCRs, the trace amine associated receptors (TAARs) [7,14]. There are 9 TAAR genes in human (including 3 pseudogenes) and 16 genes in mouse (including 1 pseudogene). The TAAR genes do not contain introns (with one exception, TAAR2 contains 1 intron) and are located next to each other on the same chromosomal segment. The phylogenetic relationship of the receptor genes, in agreement with an in-depth GPCR pharmacophore similarity comparison and pharmacological data suggest that these receptors form three distinct subfamilies [7,14]. TAAR1 is in the first subclass of four genes (TAAR1-4) highly conserved between human and rodents. TAs activate TAAR1 via Gas. Dysregulation of TAs was shown to contribute to the aetiology of various diseases like depression, psychosis, attention deficit hyperactivity disorder, substance abuse, Parkinson's disease, migraine headache, eating disorders, metabolic disorders and therefore TAAR1 ligands have a high potential for the treatment of these diseases.
Therefore, there is a broad interest to increase the knowledge about trace amine associated receptors.

### References used:

| | |
|---|---|
| 1 | Deutch, A.Y. and Roth, R.H. (1999) Neurotransmitters. In Fundamental Neuroscience (2nd edn) (Zigmond, M.J., Bloom, F.E., Landis, S.C., Roberts, J.L, and Squire, L.R., eds.), pp. 193-234, Academic Press; |
| 2 | Wong, M.L. and Licinio, J. (2001) Research and treatment approaches to depression. Nat. Rev. Neurosci. 2, 343-351; |
| 3 | Carlsson, A. et al. (2001) Interactions between monoamines, glutamate, and GABA in schizophrenia: new evidence. Annu. Rev. Pharmacol. Toxicol. 41, 237-260; |
| 4 | Tuite, P. and Riss, J. (2003) Recent developments in the pharmacological treatment of Parkinson's disease. Expert Opin. Investig. Drugs 12, 1335-1352, |
| 5 | Castellanos, F.X. and Tannock, R. (2002) Neuroscience of attention-deficit/hyperactivity disorder: the search for endophenotypes. Nat. Rev. Neurosci. 3, 617-628; |
| 6 | Usdin, Earl; Sandler, Merton; Editors. Psychopharmacology Series, Vol. 1: Trace Amines and the Brain. [Proceedings of a Study Group at the 14th Annual Meeting of the American College of Neuropsychoparmacology, San Juan, Puerto Rico] (1976); |
| 7 | Lindemann, L. and Hoener, M. (2005) A renaissance in trace amines inspired by a novel GPCR family. Trends in Pharmacol. Sci. 26, 274-28 1; |
| 8 | Branchek, T.A. and Blackburn, T.P. (2003) Trace amine receptors as targets for novel therapeutics: legend, myth and fact. Curr. Opin. Pharmacol. 3, 90-97; |
| 9 | Premont, R.T. et al. (2001) Following the trace of elusive amines. Proc. Natl. Acad. Sci. U. S. A. 98, 9474-9475; |
| 10 | Mousseau, D.D. and Butterworth, R.F. (1995) A high-affinity [3H] tryptamine binding site in human brain. Prog. Brain Res. 106, 285-291; |
| 11 | McCormack, J.K. et al. (1986) Autoradiographic localization of tryptamine binding sites in the rat and dog central nervous system. J. Neurosci. 6, 94-101; |
| 12 | Dyck, L.E. (1989) Release of some endogenous trace amines from rat striatal slices in the presence and absence of a monoamine oxidase inhibitor. Life Sci. 44, 1149-1156; |
| 13 | Parker, E.M. and Cubeddu, L.X. (1988) Comparative effects of amphetamine, phenylethylamine and related drugs on dopamine efflux, dopamine uptake and mazindol binding. J. Pharmacol. Exp. Ther. 245, 199-210; |
| 14 | Lindemann, L. et al. (2005) Trace amine associated receptors form structurally and functionally distinct subfamilies of novel G protein-coupled receptors. Genomics 85, 372-385. |

Objects of the present invention are novel compounds of formula I, their manufacture, medicaments based on a compound in accordance with the invention and their production as well as the use of compounds of formula I in the control or prevention of illnesses such as depression, anxiety disorders, bipolar disorder, attention deficit hyperactivity disorder, stress-related disorders, psychotic disorders such as schizophrenia, neurological diseases such as Parkinson's disease, neurodegenerative disorders such as Alzheimer's disease, epilepsy, migraine, hypertension, substance abuse and metabolic disorders such as eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

The preferred indications using the compounds of the present invention are depression, psychosis, Parkinson's disease, anxiety and attention deficit hyperactivity disorder (ADHD).

As used herein, the term "lower alkyl" denotes a saturated straight- or branched-chain group containing from 1 to 7 carbon atoms, for example, methyl, ethyl, propyl, isopropyl, n-butyl, i-butyl, 2-butyl, t-butyl and the like. Preferred alkyl groups are groups with 1-4 carbon atoms.

As used herein, the term "lower alkenyl" denotes a straight- or branched-chain group containing from 2 to 7 carbon atoms, wherein at least one bond is a double bond.

As used herein, the term "lower alkoxy" denotes a group wherein the alkyl residue is as defined above and which is attached via an oxygen atom.

As used herein, the term "lower alkyl substituted by halogen" denotes an alkyl group as defined above, wherein at least one hydrogen atom is replaced by halogen, for example CF₃, CHF₂, CH₂F, CH₂CF₃, CH₂CH₂CF₃, CH₂CF₂CF₃ and the like.

As used herein, the term aryl denotes an aromatic group, selected from phenyl, naphthalen-1-yl or naphthalen-2-yl.

As used herein, the term heteroaryl is an aromatic group, containing at least one O, N or S ring atom, selected from the group consisting of thiophenyl, pyridinyl, pyrimidinyl, benzofuranyl or indolyl;

The term "halogen" denotes chlorine, iodine, fluorine and bromine.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

One group of compounds are those of formula 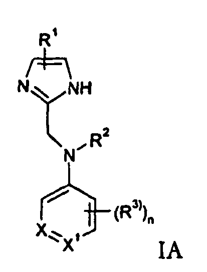 wherein
- R¹: is hydrogen or lower alkyl;
- R²: is lower alkyl, lower alkenyl, lower alkyl substituted by hydroxy,
lower alkyl substituted by halogen,
-(CH₂)ₓ-S-lower alkyl,
-(CH₂)ₓ-O-lower alkyl,
-(CH₂)ₓ-NHC(O)O-lower alkyl,
-(CH₂)ₓ-aryl or
-(CH₂)ₓ-heteroaryl;
- R³: is hydrogen, lower alkyl, lower alkoxy, halogen, hydroxy, lower alkyl substituted by halogen,
-O-(CH₂)ₘ-aryl,
-O-(CH₂)ₘ-heteroaryl,
-(CR₂)ₘ-aryl or
- (CR₂)ₘ-heteroaryl;
- R: is hydrogen, lower alkyl or hydroxy;
- X/X¹: are independently CH or N, wherein X and X¹ are not simultaneously N;
- n: is 0, 1 or 2; when n is 2, then the two R³ groups may or may not be the same;
- x: is 0, 1, 2 or 3;
- m: is 0 or 1; when m is 1, then the two R groups may or may not be the same;
and their pharmaceutically active salts, with the exception of the following known compounds:
benzyl-(*1H*-imidazol-2-ylmethyl)-phenyl-amine
ethyl-(*1H*-imidazol-2-ylmethyl)-phenyl-amine.

Preferred compounds of formula I are those, wherein Ar is phenyl. Especially preferred from this group are those, wherein R² is lower alkyl.
Such compounds are
(3-chloro-phenyl)-(1H-imidazol-2-ylmethyl)-methyl-amine
(4-chloro-phenyl)-(1H-imidazol-2-ylmethyl)-methyl-amine
(1H-imidazol-2-ylmethyl)-(3-methoxy-phenyl)-methyl-amine
(4-fluoro-phenyl)-(1H-imidazol-2-ylmethyl)-isopropyl-amine
ethyl-(3-fluoro-phenyl)-(1H-imidazol-2-ylmethyl)-amine
(3-chloro-phenyl)-(1H-imidazol-2-ylmethyl)-isopropyl-amine
(3-chloro-phenyl)-ethyl-(1H-imidazol-2-ylmethyl)-amine
(2,5-difluoro-phenyl)-(3H-imidazol-4-ylmethyl)-isopropyl-amine
(1H-imidazol-2-ylmethyl)-isopropyl-m-tolyl-amine
(3-benzyloxy-phenyl)-(1H-imidazol-2-ylmethyl)-isopropyl-amine
(1H-imidazol-2-ylmethyl)-isopropyl-[3-(pyridin-3-yloxy)-phenyl] -amine
(3-benzyl-phenyl)-(1H-imidazol-2-ylmethyl)-isopropyl-amine
biphenyl- 3-yl-(1H-imidazol-2-ylmethyl)-isopropyl-amine
[3-(4-chloro-phenoxy)-phenyl]-(1H-imidazol-2-ylmethyl)-isopropyl-amine
Ethyl-(1H-imidazol-2-ylmethyl)-(3-phenoxy-phenyl)-amine
(3-benzyloxy-phenyl)-ethyl-(1H-imidazol-2-ylmethyl)-amine or
(3,4-dichloro-phenyl)-ethyl-(1H-imidazol-2-ylmethyl)-amine.

Further preferred are compounds, wherein Ar is phenyl and R² is -(CH₂)ₓ-O-lower alkyl, for example the following compound: (1H-imidazol-2-ylmethyl)-(2-methoxy-ethyl)-phenyl-amine.
A further embodiment of the invention are compounds or formula 1, wherein Ar is pyrimidin-2-yl, pyrimidin-4-yl or pyridin-3-yl.

The present compounds of formula I and their pharmaceutically acceptable salts can be prepared by methods known in the art, for example, by processes described below, which process comprises
a) reacting a compound of formula 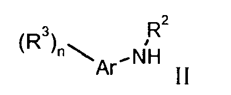 with a compound of formula 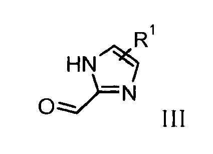 to a compound of formula 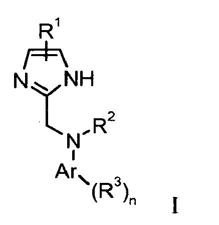 wherein R¹, R², R³, n and Ar are as defined above, or
b) reacting a compound of formula 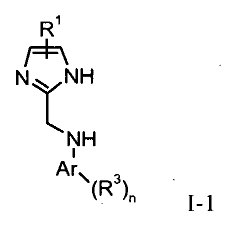 with a compound of formula R²-CHO
   to a compound of formula 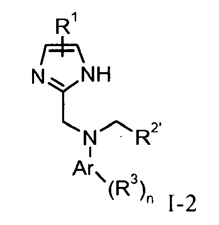 wherein R^{2'} is lower alkyl, lower alkenyl, lower alkyl substituted by hydroxy, lower alkyl substituted by halogen, -(CH₂)ₓ₋₁-S-lower alkyl, -(CH₂)ₓ₋₁-O-lower alkyl, (CH₂)ₓ₋₁-NHC(O)O-lower alkyl or (CH₂)ₓ₋₁-heteroaryl and the other substituents are as defined above, or
c) reacting a compound of formula 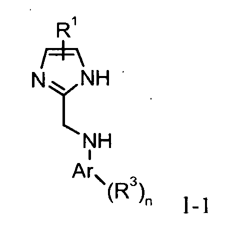 with 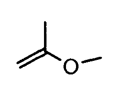 to a compound of formula 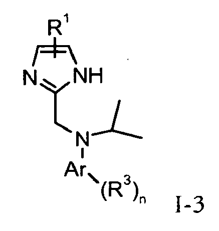 wherein the substituents are as defined above, or
d) removing a protecting group of compounds of formulas 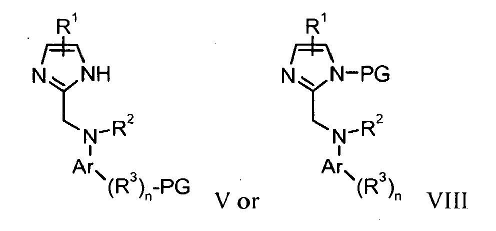 to a compound of formula 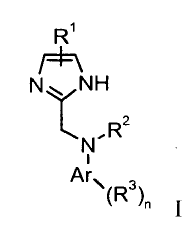 wherein the substituents are as defined above, or
e) reducing a compound of formula 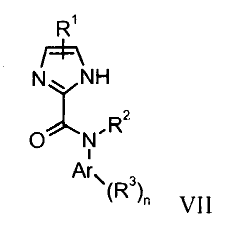 to a compound of formula 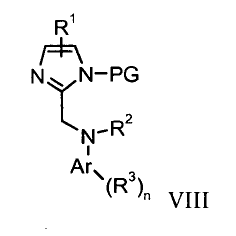 and removing the protecting group to a compound of formula I, wherein the substituents are as defined above, or
f) reacting a compound of formula 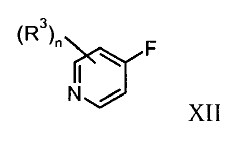 with a compound of formula 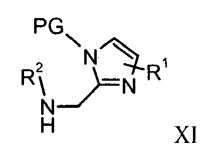 to a compound of formula 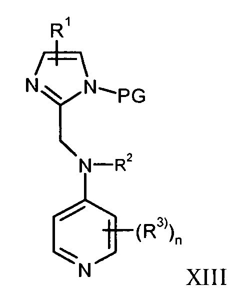 and removing the protecting group to a compound of formula 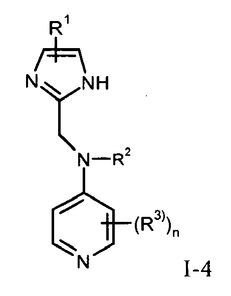 wherein the substituents are as defined above, and
if desired, converting the compounds obtained into pharmaceutically acceptable acid addition salts.

The compounds of formula I may be prepared in accordance with the process variants as described above and with the following schemes 1-5. The starting materials are either commercially available, are otherwise known in the chemical literature, or may be prepared in accordance with methods well known in the art.

### Method 1

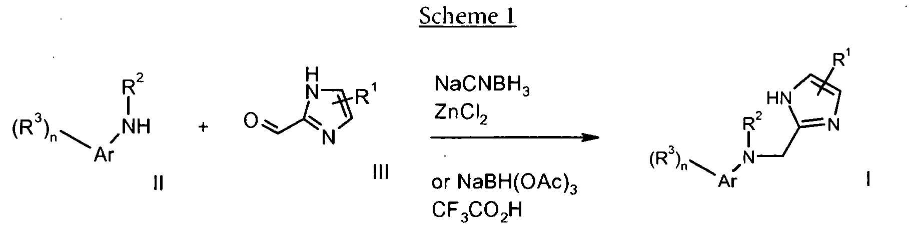

Compounds of formula I may be prepared by reductive amination using an aniline of formula II and an imidazole-2-carbaldehyde of formula III in the presence of NaCNBH₃ or NaBH(OAc)₃.

### Method 2

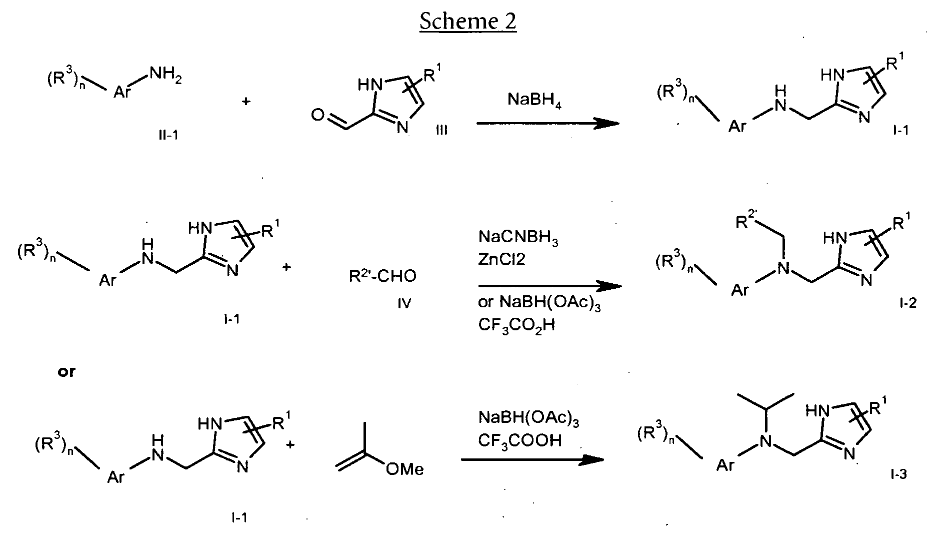

wherein R^{2'} is lower alkyl, lower alkenyl, lower alkyl substituted by hydroxy, lower alkyl substituted by halogen, (CH₂)ₓ₋₁-S-lower alkyl, -(CH₂)ₓ₋₁-O-lower alkyl, (CH₂)ₓ₋₁-NHC(O)O-lower alkyl or (CH₂)ₓ₋₁-heteroaryl.

Scheme 2 describes the preparation of compounds of formula I-1, I-2 or 1-3 by reductive amination followed by N-derivatisation.

### Method 3

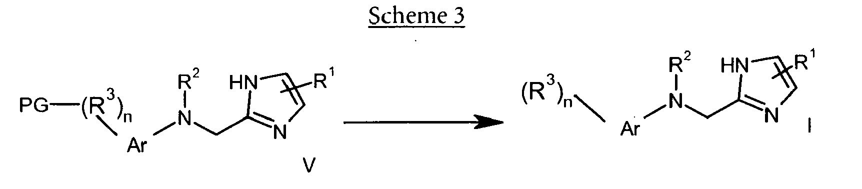

Scheme 3 describes the deprotection of a compound of formula V to a compound of formula I. The deprotection is carried out in usual matter. Compounds of formula V may be prepared according to methods 1 or 2.

### Method 4

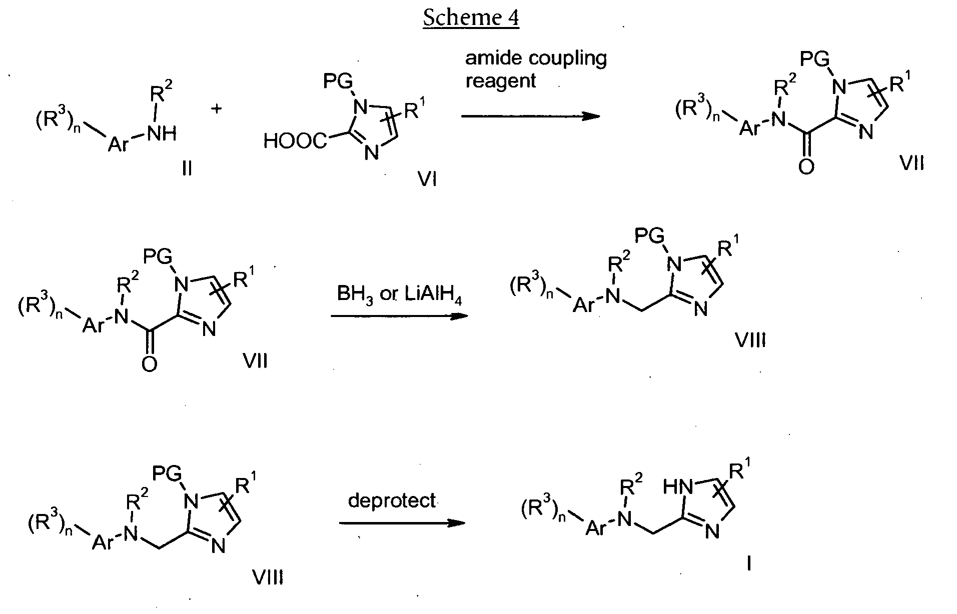

Scheme 4 describes the preparation of a compound of formula I by formation of an amide followed by reduction of the amide bond and protecting group removal.

### Method 5

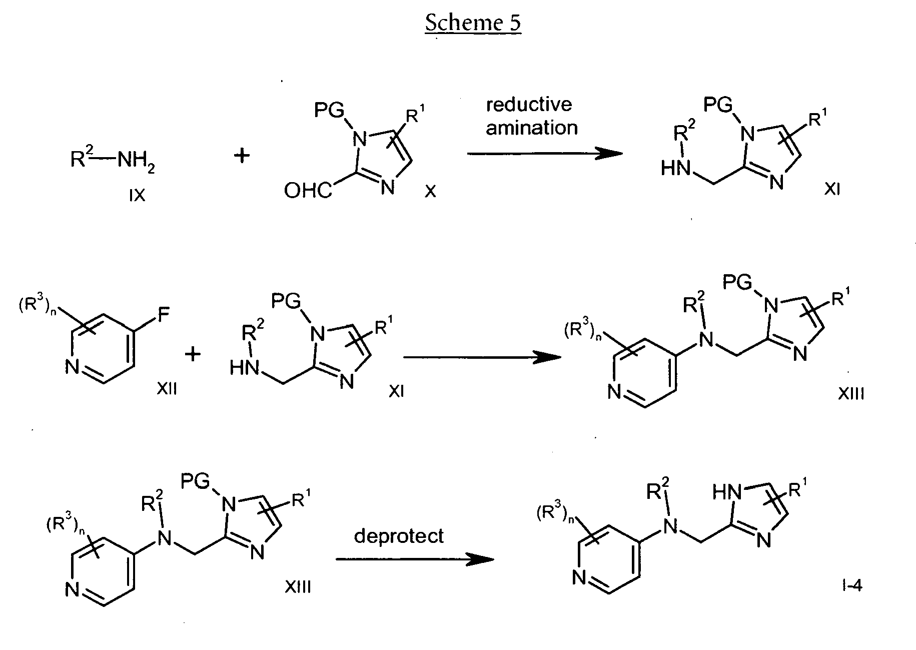

Scheme 5 describes the preparation of a compound of formula I-4 (X¹ is N) by formation of pyridine compounds by reaction of 4-fluoropyridines to protected aminomethylimidazoles.

### Isolation and purification of the compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the preparations and examples herein below. However, other equivalent separation or isolation procedures could, of course, also be used. Racemic mixtures of chiral compounds of formula I can be separated using chiral HPLC.

### Salts of compounds of formula I

The compounds of formula I are basic and may be converted to a corresponding acid addition salt. The conversion is accomplished by treatment with at least a stoichiometric amount of an appropriate acid, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained between 0 °C and 50 °C. The resulting salt precipitates spontaneously or may be brought out of solution with a less polar solvent.
The acid addition salts of the basic compounds of formula I may be converted to the corresponding free bases by treatment with at least a stoichiometric equivalent of a suitable base such as sodium or potassium hydroxide, potassium carbonate, sodium bicarbonate, ammonia, and the like.

The compounds of formula I and their pharmaceutically usable addition salts possess valuable pharmacological properties. Specifically, it has been found that the compounds of the present invention have a good affinity to the trace amine associated receptors (TAARs), especially TAAR1.
The compounds were investigated in accordance with the test given hereinafter.

### Materials and Methods

### Construction of TAAR expression plasmids and stably transfected cell lines

For the construction of expression plasmids the coding sequences of human, rat and mouse TAAR 1 were amplified from genomic DNA essentially as described by Lindemann et al. [14]. The Expand High Fidelity PCR System (Roche Diagnostics) was used with 1.5 mM Mg²⁺ and purified PCR products were cloned into pCR2.1-TOPO cloning vector (Invitrogen) following the instructions of the manufacturer. PCR products were subcloned into the pIRESneo2 vector (BD Clontech, Palo Alto, California), and expression vectors were sequence verified before introduction in cell lines.

HEK293 cells (ATCC # CRL-1573) were cultured essentially as described Lindemann et al. (2005). For the generation of stably transfected cell lines HEK293 cells were transfected with the pIRESneo2 expression plasmids containing the TAAR coding sequences (described above) with Lipofectamine 2000 (Invitrogen) according to the instructions of the manufacturer, and 24 hrs post transfection the culture medium was supplemented with 1 mg/ml G418 (Sigma, Buchs, Switzerland). After a culture period of about 10 d clones were isolated, expanded and tested for responsiveness to trace amines (all compounds purchased from Sigma) with the cAMP Biotrak Enzyme immunoassay (EIA) System (Amersham) following the non-acetylation EIA procedure provided by the manufacturer. Monoclonal cell lines which displayed a stable EC₅₀ for a culture period of 15 passages were used for all subsequent studies.

### Membrane preparation and radioligand binding

Cells at confluence were rinsed with ice-cold phosphate buffered saline without Ca²⁺ and Mg²⁺ containing 10 mM EDTA and pelleted by centrifugation at 1000 rpm for 5 min at 4 °C. The pellet was then washed twice with ice-cold phosphate buffered saline and cell pellet was frozen immediately by immersion in liquid nitrogen and stored until use at -80 °C. Cell pellet was then suspended in 20 ml HEPES-NaOH (20 mM), pH 7.4 containing 10 mM EDTA, and homogenized with a Polytron (PT 3000, Kinematica) at 10,000 rpm for 10 s. The homogenate, was centrifuged at 48,000×g for 30 min at 4 °C and the pellet resuspended in 20 ml HEPES-NaOH (20 mM), pH 7.4 containing 0.1 mM EDTA (buffer A), and homogenized with a Polytron at 10,000 rpm for 10 s. The homogenate was then centrifuged at 48,000×g for 30 min at 4 °C and the pellet resuspended in 20 ml buffer A, and homogenized with a Polytron at 10,000 rpm for 10 s. Protein concentration was determined by the method of Pierce (Rockford, IL). The homogenate was then centrifuged at 48,000×g for 10 min at 4 °C, resuspended in HEPES-NaOH (20 mM), pH 7.0 including MgCl₂ (10 mM) and CaCl₂ g protein per ml and (2 mM) (buffer B) at 200 homogenized with a Polytron at 10,000 rpm for 10 s.

Binding assay was performed at 4 °C in a final volume of 1 ml, and with an incubation time of 30 min. The radioligand [³H]-rac-2-(1,2,3,4-tetrahydro-1-naphthyl)-2-imidazoline was used at a concentration equal to the calculated *K*_{d} value of 60 nM to give a bound at around 0.1 % of the total added radioligand concentration, and a specific binding which represented approximately 70 - 80 % of the total binding. Non-specific binding was defined as the amount of [³H]-rac-2-(1,2,3,4-tetrahydro-1-naphthyl)-2-imidazoline bound in the presence of the appropriate unlabelled ligand (10µM). Competing ligands were tested in a wide range of concentrations (10 µM - 30 µM). The final dimethylsulphoxide concentration in the assay was 2%, and it did not affect radioligand binding. Each experiment was performed in duplicate. All incubations were terminated by rapid filtration through UniFilter-96 plates (Packard Instrument Company) and glass filter GF/C, pre-soaked for at least 2 h in polyethylenimine 0.3%, and using a Filtermate 96 Cell Harvester (Packard Instrument Company). The tubes and filters were then washed 3 times with 1 ml aliquots of cold buffer B. Filters were not dried and soaked in Ultima gold (45 µl/well, Packard Instrument Company) and bound radioactivity was counted by a TopCount Microplate Scintillation Counter (Packard Instrument Company).

The preferred compounds show a Ki value (µM) in mouse on TAAR1 in the range of 0.002 - 0.100 as shown in the table below.

| **Example** | **Ki (µM) mouse** | **Example** | **Ki** |
|---|---|---|---|
| 2 | 0.030 | 46 | 0.077 |
| 5 | 0.081 | 51 | 0.082 |
| 6 | 0.073 | 65 | 0.0111 |
| 10 | 0.094 | 66 | 0.0263 |
| 12 | 0.051 | 68 | 0.0331 |
| 15 | 0.050 | | |
| 21 | 0.082 | | |
| 30 | 0.036 | | |
| 37 | 0.065 | | |
| 39 | 0.089 | | |
| 41 | 0.021 | | |
| 43 | 0.025 | | |
| 45 | 0.002 | | |

The compounds of formula I and the pharmaceutically acceptable salts of the compounds of formula I can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. The administration can, however, also be effected rectally, e.g. in the form of suppositories, parenterally, e.g. in the form of injection solutions.

The compounds of formula I can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are however usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also an object of the present invention, as is a process for their production, which comprises bringing one or more compounds of formula I and/or pharmaceutically acceptable acid addition salts and, if desired, one or more other therapeutically valuable substances into a galenical administration form together with one or more therapeutically inert carriers.

The most preferred indications in accordance with the present invention are those, which include disorders of the central nervous system, for example the treatment or prevention of schizophrenia, depression, cognitive impairment and Alzheimer's disease.

The dosage can vary within wide limits and will, of course, have to be adjusted to the individual requirements in each particular case. In the case of oral administration the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

### Tablet Formulation (Wet Granulation)

| Item | Ingredients | mg/tablet | | | |
|---|---|---|---|---|---|
| | | 5 mg | 25 mg | 100 mg | 500 mg |
| 1. | Compound of formula I | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

### Manufacturing Procedure

1. Mix items 1, 2, 3 and 4 and granulate with purified water.
2. Dry the granules at 50°C.
3. Pass the granules through suitable milling equipment.
4. Add item 5 and mix for three minutes; compress on a suitable press.

### Capsule Formulation

| Item | Ingredients | mg/capsule | | | |
|---|---|---|---|---|---|
| | | 5 mg | 25 mg | 100 mg | 500 mg |
| 1. | Compound of formula I | 5 | 25 | 100 | 500 |
| 2. | Hydrous Lactose | 159 | 123 | 148 | --- |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | Total | 200 | 200 | 300 | 600 |

### Manufacturing Procedure

1. Mix items 1, 2 and 3 in a suitable mixer for 30 minutes.
2. Add items 4 and 5 and mix for 3 minutes.
3. Fill into a suitable capsule.

### Experimental

The following examples illustrate the invention but are not intended to limit its scope. Examples 11, 13, 25, 26, 61, 62, 63, 64, 71, 72, 73 and 74 are reference compounds and are not encompassed from the present scope of claims.

### Example 1

### (1H-Imidazol-2-ylmethyl)-isopropyl-phenyl-amine

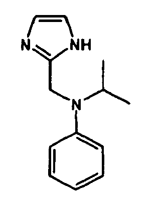

### a) (1H-Imidazol-2-ylmethyl)-phenyl-amine

To a solution of aniline (0.50 g, 5.37 mmol) in methanol (7 ml) was added imidazole-2-carboxyaldehyde (0.62 g, 6.45 mmol). After stirring the mixture overnight at room temperature sodium borohydride (0.305 g, 8.05 mmol) was added. The reaction mixture was stirred at room temperature for 4 hours. Then water was added and the mixture was extracted with ethyl acetate. The organic layer was separated, washed with water, dried over magnesium sulfate and evaporated. The residue was purified by crystallization (ethyl acetate) to yield a white solid (0.71 g, 76%); MS (ISP): 174.1 ((M+H)⁺˙).

### b) (1H-Imidazol-2-ylmethyl)-isopropyl-phenyl-amine

To a solution of (1H-imidazol-2-ylmethyl)-phenyl-amine (0.71 g, 4.1 mmol) in 1,2-dichloroethane (10 ml) were added successively 2-methoxypropene (0.5 ml, 5.3 mmol), trifluoroacetic acid (0.47ml, 6.1 mmol) and sodium triacetoxyborohydride (1.3 g, 6.1 mmol). After stirring the mixture overnight at room temperature water and ethyl acetate were added. After extracting the aqueous phase twice with ethyl acetate the combined organic layers were dried over magnesium sulfate and evaporated. The residue was purified by column chromatography (SiO2, heptane/ethyl acetate=1:1) to yield a white solid (0.335 mg, 38%); MS (ISP): 216.2 ((M+H)⁺˙).

### Example 2

### (3-Chloro-phenyl)-(1H-imidazol-2-ylmethyl)-methyl-amine

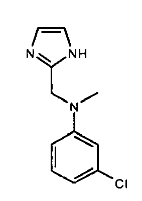

To a solution of 3-chloro-N-methylaniline (0.283 g, 2 mmol) in 1,2-dichloroethane (10 ml) were added molecular sieves (2g, size 0.4 nM) and imidazol-2-carboxyaldehyde (0.288 g, 3 mmol). After stirring the mixture for 5 min at room temperature sodium triacetoxyborohydride (0.848 g, 4 mmol) and acetic acid (5 drops) were added. The reaction mixture was stirred at room temperature overnight. For workup dichloromethane (50 ml) and 1M sodium bicarbonate solution (30 ml) were added and the mixture was shaken. The organic layer was separated, dried over magnesium sulfate and evaporated. The residue was purified using flash chromatography (column: Isolute^{®} Flash-NH₂ (Separtis); eluent: ethyl acetate/methanol = 95:5) to yield a white solid (0.13g, 29%); MS (ISP): 222.1 ((M+H)⁺˙).

### Example 3

### 2-[(1H-Imidazol-2-ylmethyl)-phenyl-amino]-ethanol

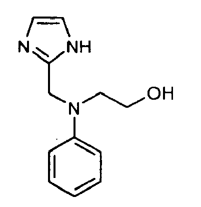

The title compound, MS (ISP): 218.2 ((M+H^{+.}) was obtained in comparable yield analogous to the procedure described for Example 2 using N-(2-hydroxyethyl)-aniline instead of 3-chloro-N-methylaniline.

### Example 4

### (1H-Imidazol-2-ylmethyl)-(4-methoxy-phenyl)-methyl-amine

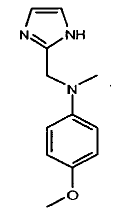

The title compound, MS (EI): 218.4 (M^{+.}) was obtained in comparable yield analogous to the procedure described for Example 2 using N-methyl-4-methoxyaniline instead of 3-chloro-N-methylaniline.

### Example 5

### (4-Chloro-phenyl)-(1H-imidazol-2-ylmethyl)-methyl-amine

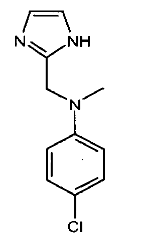

The title compound, MS (ISP): 222.1 ((M+H)^{+.}) was obtained in comparable yield analogous to the procedure described for Example 2 using N-methyl-p-chloroaniline instead of 3-chloro-N-methylaniline.

### Example 6

### (1H-Imidazol-2-ylmethyl)-(3-methoxy-phenyl)-methyl-amine

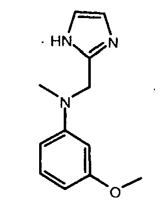

### a) 1-Benzyl-1H-imidazole-2-carboxylic acid (3-methoxy-phenyl)-methyl-amide

N-Methyl-3-methoxyaniline (0.302 g, 2.0 mmol) was dissolved in acetonitrile (10 ml). Then 1-benzyl-2-imidazolecarboxylic acid (0.302 g, 2.2 mmol), N-ethyldiisopropylamine (0.775 g, 6 mmol), 2-(1H-benzotriazol-1-yl)-1,1,3,3-tetramethyluronium tetrafluoroborate (TBTU; 1.0 g, 3.1 mmol) and for complete dissolution 2 ml of dimethylformamide were added. The reaction mixture was stirred overnight at room temperature. For workup acetonitrile was evaporated in vacuo, sodium bicarbonate solution was added and the mixture was extracted twice with dichloromethane. The combined organic layers were dried over magnesium sulfate and evaporated. The residue was purified using chromatography (SiO₂; eluent: dichloromethane/ methanol = 97:3) to yield a light yellow oil (0.485g, 75 %); MS (ISP): 322.2 ((M+H)^{+.}).

### b) (1-Benzyl-1H-imidazol-2-ylmethyl)-(3-methoxy-phenyl)-methyl-amine

A solution of 1-benzyl-1H-imidazole-2-carboxylic acid (3-methoxy-phenyl)-methylamide (0.20 g, 0.64 mmol) was dissolved in tetrahydrofuran (5 ml). Then boranetetrahydrofuran solution (3.6 ml, 1M, 3.6 mmol) was added at 0°C and the reaction mixture was heated in a sealed tube for 4 hours. For workup hydrochloric acid (1M) was added until gas evolution stopped. Then the organic solvent was evaporated, more hydrochloric acid (3 ml, 1M) was added and the mixture was heated to 100°C for 1 hour. After cooling, ammonium hydroxide solution (25%) was added until basic pH and the mixture was extracted with dichloromethane. The combined organic layers were dried over magnesium sulfate and evaporated. The residue was purified by flash chromatography (SiO2: heptane/ethyl acetate = 1:1) to yield a light yellow oil (0.086 g, 44%); MS (ISP): 308.1 ((M+H)^{+.}).

### c) (1H-Imidazol-2-ylmethyl)-(3-methoxy-phenyl)-methyl-amine

(1-Benzyl-1H-imidazol-2-ylmethyl)-(3-methoxy-phenyl)-methyl-amine (0.077 g, 0.25 mmol) was dissolved in ethanol (5 ml), acetic acid (0.075 g, 1.25 mmol) and palladium on charcoal (15 mg, 10% Pd) were added and the mixture was hydrogenated at 60°C for 90 minutes. The catalyst was filtered off using Celite. To obtain the free base (remove acetic acid) the solution was put onto a SCX-column (0.5 g from Varian, sulfonic acid modified silica gel). After washing the SCX column with methanol (1 ml, discarded) the product was liberated from the column by washing with ammonia in methanol (2 ml, 1M). The solvent was evaporated and the residue was purified using column filtration (SiO2; dichloromethane/methanol = 95:5) to yield an off-white solid (0.04 g, 74 %); MS (ISP): 218.0 ((M+H)^{+.}).

### Example 7

### Isopropyl-(4-methyl-1H-imidazol-2-ylmethyl)-phenyl-amine

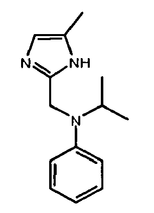

The title compound, MS (ISP): 230.1 ((M+H)^{+.}) was obtained in comparable yield analogous to the procedure described for Example 2 using isopropyl-phenyl-amine instead of 3-chloro-N-methylaniline and 4-methyl-imidazol-2-carboxyaldehyde instead of imidazol-2-carboxyaldehyde.

### Example 8

### (3-Fluoro-phenyl)-(1H-imidazol-2-ylmethyl)-isopropyl-amine

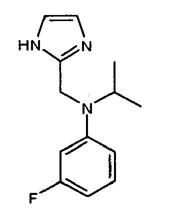

The title compound, MS (ISP): 234.3 ((M+H^{+.}) was obtained in comparable yield analogous to the procedure described for Example 1 using 3-fluoroaniline instead of aniline in step a).

### Example 9

### (2-Fluoro-phenyl)-(1H-imidazol-2-ylmethyl)-isopropyl-amine

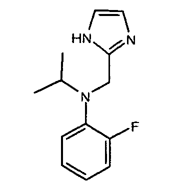

### a) 1-Benzyl-1H-imidazole-2-carboxylic acid (2-fluoro-phenyl)-isopropyl-amide

A mixture of 1-benzyl-2-imidazolecarboxylic acid (0.624 g, 3.0 mmol) and dichloromethylene-dimethyliminium chloride (0.487g, 3.0 mmol) in dichloromethane (15 ml) was stirred at room temperature for 2 hours. Then N-isopropyl-2-fluoroaniline (0.306 g, 2.0 mmol) and sodium bicarbonate (0.840 g, 10 mmol) were added and stirring was continued overnight. For workup water was added and the mixture was extracted twice with dichloromethane. The combined organic layers were dried over magnesium sulfate and evaporated. The residue was purified by chromatography (SiO₂; eluent: heptane/ ethyl acetate = 2:1) to yield a colorless oil (0.268 g, 40 %); MS (ISP): 338.3 ((M+H)^{+.}).

### b) (2-Fluoro-phenyl)-(1H-imidazol-2-ylmethyl)-isopropyl-amine

The title compound, MS (ISP): 234.1 ((M+H)^{+.}) was obtained in comparable yield analogous to the procedure described for Example 6 b) and c) using 1-benzyl-1H-imidazole-2-carboxylic acid (2-fluoro-phenyl)-isopropyl-amide instead of 1-benzyl-1H-imidazole-2-carboxylic acid (3-methoxy-phenyl)-methyl-amide in step b).

### Example 10

### (4-Fluoro-phenyl)-(1H-imidazol-2-ylmethyl)-isopropyl-amine

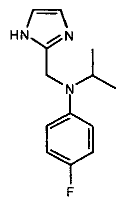

The title compound, MS (ISP): 234.0 ((M+H)^{+.}) was obtained in comparable yield analogous to the procedure described for Example 1 using 4-fluoroaniline instead of aniline in step a).

### Example 11

### (1H-Imidazol-2-ylmethyl)-(2-isopropyl-6-methyl-phenyl)-amine

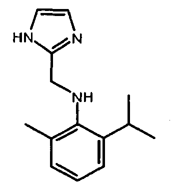

To a solution of 2-isopropyl-6-methyl-aniline (1.49 g, 10 mmol) in methanol (10 ml) was added imidazole-2-carboxyaldehyde (0.96 g, 10 mmol). After stirring the mixture overnight at 60°C sodium borohydride (0.567 g, 15 mmol) were added. The reaction mixture was stirred at room temperature for 4 hours. Then water was added and the mixture was extracted with ethyl acetate. The organic layer was separated, washed with water, dried over magnesium sulfate and evaporated. The residue was purified by chromatography (column: Isolate^{®} Flash-NH₂ (Separtis); eluent: heptane/ethyl acetate = 1:1) to yield a white solid (1.29g, 56%); MS (ISP): 230.1 ((M+H)^{+.}).

### Example 12

### Ethyl-(3-fluoro-phenyl)-(1H-imidazol-2-ylmethyl)-amine

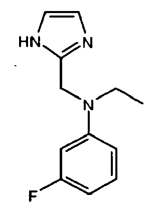

### a) (1H-Imidazol-2-ylmethyl)-(3-fluoro-phenyl)-amine

To a solution of 3-fluoroaniline (0.33 g, 3.0 mmol) in methanol (7 ml) was added imidazole-2-carboxyaldehyde (0.29 g, 3.0 mmol) and the mixture was stirred overnight at 60°C. After cooling sodium borohydride (0.17 g, 4.5 mmol) was added and the reaction mixture was stirred at room temperature for 4 hours. Then water was added and the mixture was extracted with ethyl acetate. The organic layer was separated, washed with water, dried over magnesium sulfate and evaporated. The residue was purified by chromatography (SiO2, ethyl acetate) to yield a light yellow solid (0.315 g, 55%); MS (ISP): 192.1 ((M+H)^{+.}).

### b) Ethyl-(3-fluoro-phenyl)-(1H-imidazol-2-ylmethyl)-amine

(1H-Imidazol-2-ylmethyl)-(3-fluoro-phenyl)-amine (0.19 g, 1 mmol) was dissolved in methanol (15 ml). Then acetaldehyde (0.28 ml, 5 mmol), zinc chloride (0.55 g, 4 mmol) and sodium cyanoborohydride (0.31 g, 5 mmol) were added and the reaction mixture was allowed to stir at 40°C overnight. After cooling, the reaction mixture was poured onto ammoniumchloride/ice and extracted with ethyl acetate (2 times 50 ml). The organic layer was dried over magnesium sulfate and evaporated. The residue was purified using flash chromatography (SiO₂; eluent: heptane/ ethyl acetate = 90: 10) to yield an off-white solid (0.118 g, 54 %); MS (ISP): 220.2 ((M+H)^{+.}).

### Example 13

### (2-Chloro-phenyl)-(1H-imidazol-2-ylmethyl)-amine

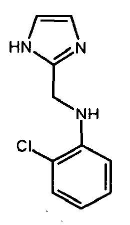

The title compound, MS (ISP): 208.1 ((M+H)^{+.}) was obtained in comparable yield analogous to the procedure described for Example 11 using 2-chloroaniline instead of 2-isopropyl-6-methyl-aniline.

### Example 14

### (2-Chloro-phenyl)-(1H-imidazol-2-ylmethyl)-isopropyl-amine

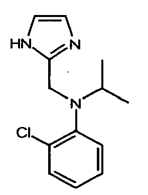

The title compound, MS (ISP): 250.1 ((M+H)^{+.}) was obtained in comparable yield analogous to the procedure described for Example 1 using 2-chloroaniline instead of aniline in step a).

### Example 15

### (3-Chloro-phenyl)-(1H-imidazol-2-ylmethyl)-isopropyl-amine

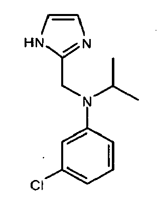

The title compound, MS (1SP): 250.3 ((M+H)^{+.}) was obtained in comparable yield analogous to the procedure described for Example 1 using 3-chloroaniline instead of aniline in step a).

### Example 16

### (4-Chloro-phenyl)-(1H-imidazol-2-ylmethyl)-isopropyl-amine

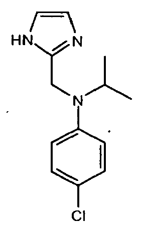

The title compound, MS (ISP): 250.1 ((M+H)^{+.}) was obtained in comparable yield analogous to the procedure described for Example 1 using 4-chloroaniline instead of aniline in step a).

### Example 17

### (1H-Imidazol-2-ylmethyl)-isopropyl-(2-methoxy-phenyl)-amine

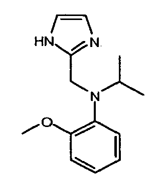

The title compound, MS (ISP): 246.4 ((M+H)^{+.}) was obtained in comparable yield analogous to the procedure described for Example 1 using 2-methoxyaniline instead of aniline in step a).

### Example 18

### (1H-Imidazol-2-ylmethyl)-(3-methylsulfanyl-propyl)-phenyl-amine

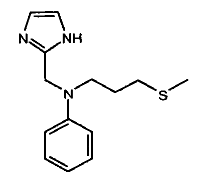

The title compound, MS (ISP): 262.1 ((M+H)^{+.}) was obtained in comparable yield analogous to the procedure described for Example 12 using aniline instead of 3-fluoroaniline in step a) and 3-(methylthio)-propionaldehyde instead of acetaldehyde in step b).

### Example 19

### (1H-Imidazol-2-ylmethyl)-isobutyl-phenyl-amine

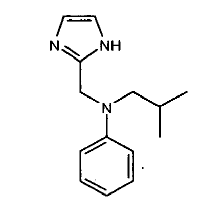

The title compound, MS (ISP): 230.3 ((M+H)^{+.}) was obtained in comparable yield analogous to the procedure described for Example 12 using aniline instead of 3-fluoroaniline in step a) and isobutyraldehyde instead of acetaldehyde in step b).

### Example 20

### (1H-Imidazol-2-ylmethyl)-(3-methyl-but-2-enyl)-phenyl-amine; 1:1 mixture with (1H-imidazol-2-ylmethyl)-(3-methyl-butyl)-phenyl-amine

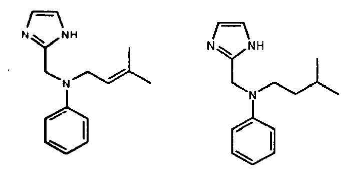

The title compound, MS (ISP): 242.1; 244.4 ((M+H)^{+.}) was obtained in comparable yield analogous to the procedure described for Example 12 using aniline instead of 3-fluoroaniline in step a) and 3-methylcrotonaldehyde instead of acetaldehyde in step b).

### Example 21

### (1H-Imidazol-2-ylmethyl)-(2-methoxy-ethyl)-phenyl-amine

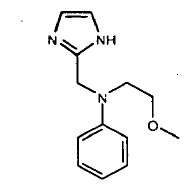

The title compound, MS (ISP): 232.3 ((M+H)^{+.}) was obtained in comparable yield analogous to the procedure described for Example 12 using aniline instead of 3-fluoroaniline in step a) and methoxyacetaldehyde instead of acetaldehyde in step b).

### Example 22

### (1H-Imidazol-2-ylmethyl)-phenyl-(3,3,3-trifluoro-propyl)-amine

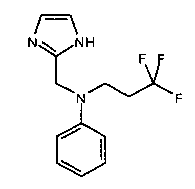

The title compound, MS (ISP): 270.4 ((M+H)^{+.}) was obtained analogous to the procedure described for Example 12 using aniline instead of 3-fluoroaniline in step a) and 3,3,3-trifluoropropionaldehyde instead of acetaldehyde in step b).

### Example 23

### {2-[(1H-Imidazol-2-ylmethyl)-phenyl-amino]-ethyl}-carbamic acid tert-butyl ester

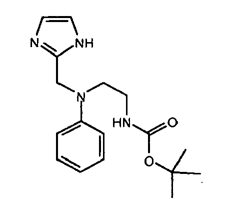

The title compound, MS (ISP): 317.4 ((M+H)^{+.}) was obtained in comparable yield analogous to the procedure described for Example 12 using using aniline instead of 3-fluoroaniline in step a) and tert-butyl N-(2-oxoethyl)carbamate instead of acetaldehyde in step b).

### Example 24

### (1H-Imidazol-2-ylmethyl)-phenyl-thiophen-3-ylmethyl-amine

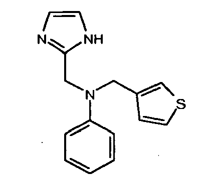

The title compound, MS (ISP): 270.3 ((M+H)^{+.}) was obtained in comparable yield analogous to the procedure described for Example 12 using aniline instead of 3-fluoroaniline in step a) and 3-thiophenecarboxyaldehyde instead of acetaldehyde in step b).

### Example 25

### (1H-Imidazol-2-ylmethyl)-(3-methoxy-phenyl)-amine

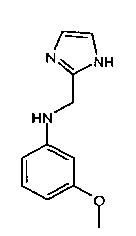

The title compound, MS (ISP): 203.9 ((M+H)^{+.}) was obtained in comparable yield analogous to the procedure described for Example 11 using 3-methoxyaniline instead of 2-isopropyl-6-methyl-aniline.

### Example 26

### (2-Fluoro-phenyl)-(1H-imidazol-2-ylmethyl)-amine

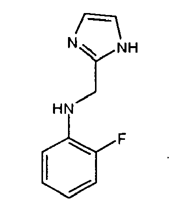

The title compound, MS (ISP): 192.0 ((M+H)^{+.}) was obtained in comparable yield analogous to the procedure described for Example 11 using 2-fluoroaniline instead of 2-isopropyl-6-methyl-aniline.

### Example 27

### Ethyl-(1H-imidazol-2-ylmethyl)-(3-methoxy-phenyl)-amine

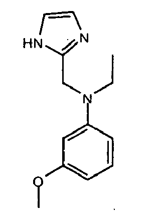

The title compound, MS (ISP): 231.9 ((M+H)^{+.}) was obtained in comparable yield analogous to the procedure described for Example 12 using using 3-methoxyaniline instead of 3-fluoroaniline in step a).

### Example 28

### Ethyl-(2-fluoro-phenyl)-(1H-imidazol-2-ylmethyl)-amine

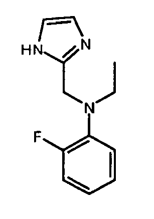

The title compound, MS (ISP): 220.1 ((M+H)^{+.}) was obtained in comparable yield analogous to the procedure described for Example 12 using using 2-fluoroaniline instead of 3-fluoroaniline in step a).

### Example 29

### Ethyl-(4-fluoro-phenyl)-(1H-imidazol-2-ylmethyl)-amine

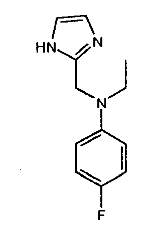

The title compound, MS (ISP): 220.2 ((M+H)^{+.}) was obtained in comparable yield analogous to the procedure described for Example 12 using using 4-fluoroaniline instead of 3-fluoroaniline in step a).

### Example 30

### (3-Chloro-phenyl)-ethyl-(1H-imidazol-2-ylmethyl)-amine

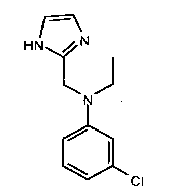

The title compound, MS (ISP): 235.9 ((M+H)^{+.}) was obtained in comparable yield analogous to the procedure described for Example 12 using using 3-chloroaniline instead of 3-fluoroaniline in step a).

### Example 31

### (1H-Imidazol-2-ylmethyl)-(2-methoxy-phenyl)-methyl-amine

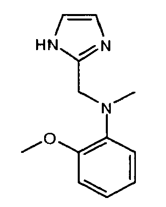

The title compound, MS (ISP): 218.4 ((M+H)^{+.}) was obtained in comparable yield analogous to the procedure described for Example 9 using 2-methoxy-N-methylaniline instead of N-isopropyl-2-fluoroaniline in step a).

### Example 32

### (1H-Imidazol-2-ylmethyl)-methyl-phenyl-amine

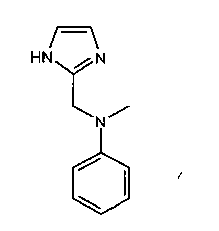

The title compound, MS (ISP): 188.3 ((M+H)^{+.}) was obtained in comparable yield analogous to the procedure described for Example 9 using N-methylaniline instead of N-isopropyl-2-fluoroaniline in step a).

### Example 33

### (4-Fluoro-phenyl)-(1H-imidazol-2-ylmethyl)-methyl-amine

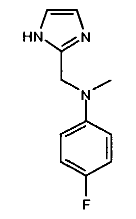

The title compound, MS (ISP): 206.1 ((M+H)^{+.}) was obtained in comparable yield analogous to the procedure described for Example 12 using 4-fluoroaniline instead of 3-fluoroaniline in step a) and formaldehyde instead of acetaldehyde in step b).

### Example 34

### (6-Chloro-pyridin-3-yl)-ethyl-(1H-imidazol-2-ylmethyl)-amine

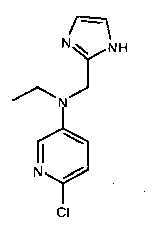

The title compound, MS (ISP): 208.6; 210.9 ((M+H)^{+.}) was obtained in comparable yield analogous to the procedure described for Example 12 using 5-amino-2-chloropyridine instead of 3-fluoroaniline in step a).

### Example 35

### (1H-Imidazol-2-ylmethyl)-isopropyl-(3-methoxy-phenyl)-amine

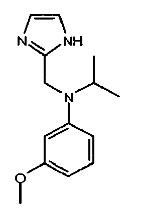

The title compound, MS (ISP): 246.3 ((M+H)^{+.}) was obtained in comparable yield analogous to the procedure described for Example 1 using 3-methoxyaniline instead of aniline in step a).

### Example 36

### (1H-Imidazol-2-ylmethyl)-isopropyl-(3-trifluoromethoxy-phenyl)-amine

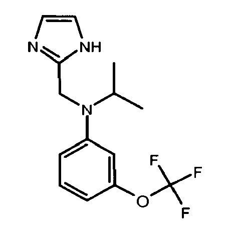

The title compound, MS (ISP): 300.1 ((M+H)^{+.}) was obtained analogous to the procedure described for Example 1 using 3-trifluoromethoxy-aniline instead of aniline in step a).

### Example 37

### (2,5-Difluoro-phenyl)-(3H-imidazol-4-ylmethyl)-isopropyl-amine

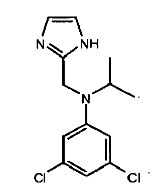

The title compound, MS (ISP): 284.2; 286.1 ((M+H)^{+.}) was obtained analogous to the procedure described for Example 1 using 3,5-dichloroaniline instead of aniline in step a).

### Example 38

### (3-Chloro-5-fluoro-phenyl)-(iH-imidazol-2-ylmethyl)-isopropyl-amine

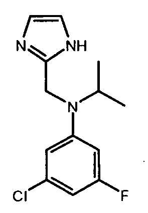

The title compound, MS (ISP): 268.2 ((M+H)^{+.}) was obtained in comparable yield analogous to the procedure described for Example 1 using 3-chloro-5-fluoroaniline instead of aniline in step a).

### Example 39

### (1H-Imidazol-2-ylmethyl)-isopropyl-m-tolyl-amine

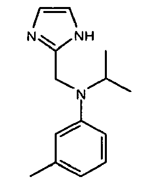

The title compound, MS (ISP): 230.3 ((M+H)^{+.}) was obtained in comparable yield analogous to the procedure described for Example 1 using 3-methylaniline instead of aniline in step a).

### Example 40

### (4-Fluoro-3-methoxy-phenyl)-(1H-imidazol-2-ylmethyl)-isopropyl-amine

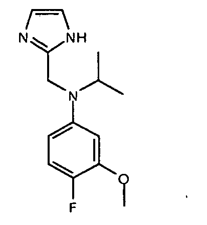

The title compound, MS (ISP): 264.0 ((M+H)^{+.}) was obtained in comparable yield analogous to the procedure described for Example 1 using 4-fluoro-3-methoxyaniline instead of aniline in step a).

### Example 41

### (3-Benzyloxy-phenyl)-(1H-imidazol-2-ylmethyl)-isopropyl-amine

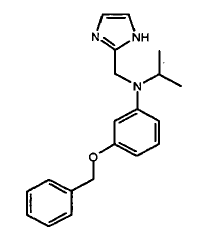

The title compound, MS (ISP): 322.3 ((M+H)^{+.}) was obtained in comparable yield analogous to the procedure described for Example 1 using 3-benzyloxyaniline instead of aniline in step a).

### Example 42

### (3-Chloro-5-trifluoromethyl-phenyl)-(1H-imidazol-2-ylmethyl)-isopropyl-amine

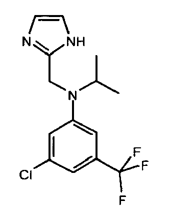

The title compound, MS (ISP): 318.1 ((M+H)^{+.}) was obtained in comparable yield analogous to the procedure described for Example 1 using 3-chloro-5-trifluoromethylaniline instead of aniline in step a).

### Example 43

### (1H-Imidazol-2-ylmethyl)-isopropyl-[3-(pyridin-3-yloxy)-phenyl]-amine

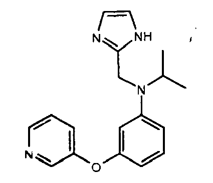

The title compound, MS (ISP): 308.4 ((M+H)^{+.}) was obtained in comparable yield analogous to the procedure described for Example 1 using 3-(pyridin-3-yloxy)-phenyl-amine instead of aniline in step a).

### Example 44

### (1H-Imidazol-2-ylmethyl)-isopropyl-(3-phenoxy-phenyl)-amine

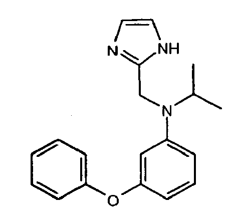

The title compound, MS (ISP): 309.3 ((M+H)^{+.}) was obtained in comparable yield analogous to the procedure described for Example 1 using 3-phenoxy-aniline instead of aniline in step a).

### Example 45

### (3-Benzyl-phenyl)-(1H-imidazol-2-ylmethyl)-isopropyl-amine

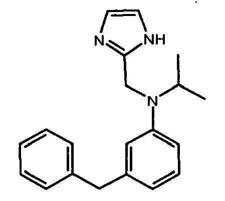

The title compound, MS (ISP): 306.5 ((M+H)^{+.}) was obtained in comparable yield analogous to the procedure described for Example 1 using 3-benzylaniline instead of aniline in step a).

### Example 46

### Biphenyl-3-yl-(1H-imidazol-2-ylmethyl)-isopropyl-amine

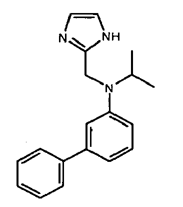

The title compound, MS (ISP): 292.3 ((M+H)^{+.}) was obtained in comparable yield analogous to the procedure described for Example 1 using biphenyl-3-ylamine instead of aniline in step a).

### Example 47

### Biphenyl-3-yl-(1H-imidazol-2-ylmethyl)-isopropyl-amine

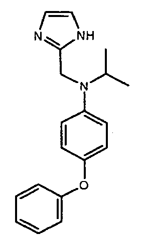

The title compound, MS (ISP): 308.5 ((M+H)^{+.}) was obtained in comparable yield analogous to the procedure described for Example 1 using 4-phenoxy-aniline instead of aniline in step a).

### Example 48

### [4-(3,4-Dichloro-phenoxy)-phenyl]-(1H-imidazol-2-ylmethyl)-isopropyl-amine

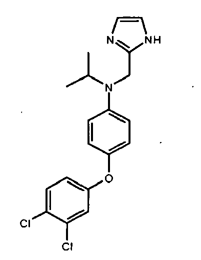

The title compound, MS (ISP): 376.1; 378.2 ((M+H)^{+.}) was obtained in comparable yield analogous to the procedure described for Example 1 using 4-(3',4'-dichlorophenoxy)-aniline instead of aniline in step a).

### Example 49

### (1H-Imidazol-2-ylmethyl)-isopropyl-[4-(4-tritluoromethyl-phenoxy)-phenyl]-amine

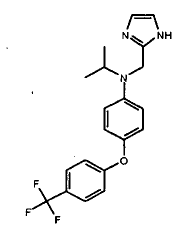

The title compound, MS (ISP): 376.2 ((M+H)^{+.}) was obtained in comparable yield analogous to the procedure described for Example 1 using 4-(4-trifluoromethylphenoxy)-aniline instead of aniline in step a).

### Example 50

### (1H-lmidazol-2-ylmethyl)-isopropyl-[4-(4-methoxy-phenoxy)-phenyl]-amine

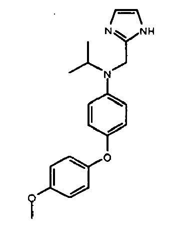

The title compound, MS (ISP): 338.2 ((M+H)^{+.}) was obtained in comparable yield analogous to the procedure described for Example 1 using 4-(4-methoxy-phenoxy)-aniline instead of aniline in step a).

### Example 51

### [3-(4-Chloro-phenoxy)-phenyl]-(1H-imidazol-2-ylmethyl)-isopropyl-amine

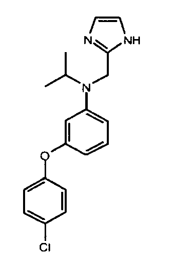

The title compound, MS (1SP): 342.1; 344.1 ((M+H)^{+.}) was obtained in comparable yield analogous to the procedure described for Example 1 using 4-(4-chloro-phenoxy)-aniline instead of aniline in step a).

### Example 52

### (2,6-Difluoro-pyridin-4-yl)-ethyl-(1H-imidazol-2-ylmethyl)-amine

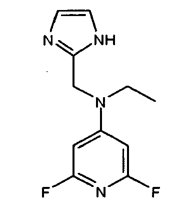

### a) Ethyl-[1-(2-trimethylsilanyl-ethoxymethyl)-1H-imidazol-2-ylmethyl]-amine

To a saturated solution of ethylamine in methanol (3 ml) was added 1-(2-trimethylsilyl)ethoxymethyl-2-imidazolecarboxaldehyde (0.2 g, 0.88 mmol) and the mixture was stirred for 1 hour. Sodium borohydride (0.05 g, 1.3 mmol) was added and the mixture was stirred overnight at 50°C.
Water was added and the solution was extracted three times with ethyl acetate. The combined organic layers were dried over magnesium sulfate and evaporated. The residue was purified by flash chromatography (SiO2: ethyl acetate/methanol = 9:1) to yield a yellow oil (0.176 g, 78%); MS (ISP): 256.0 ((M+H)^{+.}).

### b) (2,6-Difluoro-pyridin-4-yl)-ethyl-(1H-imidazol-2-ylmethyl)-amine

A mixture of ethyl-[[1-(2-trimethylsilanyl-ethoxymethyl)-1H-imidazol-2-ylmethyl]-amine (0.1 g, 0.39 mmol) and 2,4,6-trifluoropyridine (0.2 g; 1.5 mmol) was heated in a sealed vessel in a microwave oven for 1.5 h at 170°C. Then water and dichloromethane was added and the organic layer was separated, dried over magnesium sulfate and evaporated. To the residue tetrabutylammonium fluoride solution in tetrahydrofuran (1M, 1 ml, 1 mmol) was added and the mixture was stirred overnight. The solvent was evaporated and the residue was purified by flash chromatography (column: Isolute^{®} Flash-NH₂ from Separtis; eluent: heptane/ethyl acetate=1:1) to yield a white solid, (0.01 g, 10%); MS (ISP): 239.0 ((M+H)^{+.}).

### Example 53

### {3-[(1H-imidazol-2-ylmethyl)-isopropyl-amino]-phenyl}-phenyl-methanol

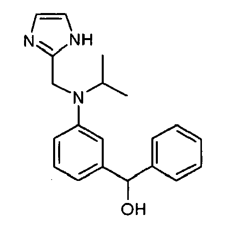

The title compound, MS (ISP): 322.4 ((M+H)^{+.}) was obtained in comparable yield analogous to the procedure described for Example 1 using 3-aminobenzophenone instead of aniline in step a).

### Example 54

### 3-[(1H-Imidazol-2-ylmethyl)-isopropyl-amino]-phenol

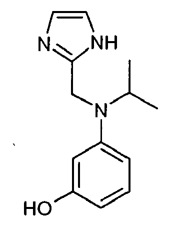

(3-Benzyloxy-phenyl)-(3H-imidazol-2-ylmethyl)-isopropyl-amine (0.285 g, 0.89 mmol) was dissolved in ethanol (5 ml), palladium on charcoal (30 mg, 10% Pd) was added and the mixture was hydrogenated for 5 hours at room temperature. The catalyst was filtered off and the solvent was evaporated. The residue was purified by flash chromatography (column: Isolute^{®} Flash-NH₂ from Separtis; eluent: ethyl acetate) to yield a white foam, (0.162 g, 79 %); MS (ISP): 232.1 ((M+H^{+.}).

### Example 55

### (1H-Imidazol-2-ylmethyl)-isopropyl-[3-(pyridin-4-yloxy)-phenyl]-amine

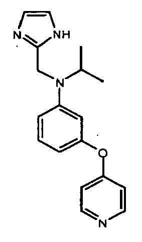

The title compound, MS (ISP): 309.3 ((M+H)^{+.}) was obtained in comparable yield analogous to the procedure described for Example 1 using 3-(pyridin-4-yloxy)-phenyl-amine instead of aniline in step a).

### Example 56

### (1H-Imidazol-2-ylmethyl)-isopropyl-(3-pyrimidin-5-yl-phenyl)-amine

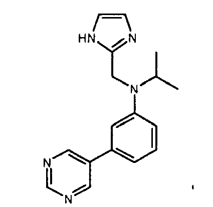

The title compound, MS (ISP): 294.0 ((M+H)^{+.}) was obtained in comparable yield analogous to the procedure described for Example 1 using (3-pyrimidin-5-yl-phenyl)-amine instead of aniline in step a).

### Example 57

### Ethyl-(1H-imidazol-2-ylmethyl)-(4-methoxy-pyrimidin-2-yl)-amine

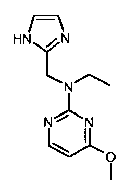

The title compound, MS (ISP): 234.1 ((M+H)^{+.}) was obtained in comparable yield analogous to the procedure described for Example 52 using 2-chloro-4-methoxypyrimidine instead of 2,4,6-trifluoropyridine in step b).

### Example 58

### (2-Benzyl-6-chloro-pyrimidin-4-yl)-ethyl-(1H-imidazol-2-ylmethyl)-amine

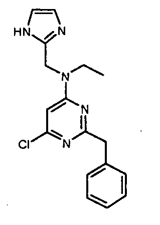

The title compound, MS (ISP): 328.2 ((M+H)^{+.}) was obtained in comparable yield analogous to the procedure described for Example 52 using 2-benzyl-4,6-dichloropyrimidine instead of 2,4,6-trifluoropyridine in step b).

### Example 59

### (2-Benzyl-pyrimidin-4-yl)-ethyl-(1H-imidazol-2-ylmethyl)-amine

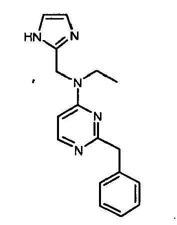

(2-Benzyl-6-chloro-pyrimidin-4-yl)-ethyl-(1H-imidazol-2-ylmethyl)-amine (0.164 g, 0.5 mmol) was dissolved in methanol (5 ml), ammonium formate (0.315 g, 0.5 mmol) and palladium on charcoal (0.164 g, 10% Pd) was added and the mixture was refluxed for one hour. After cooling the catalyst was filtered off and the solvent was evaporated. The residue was purified by flash chromatography (column: Isolute^{®} Flash-NH₂ from Separtis; eluent: ethyl acetate / methanol = 95:5) to yield a white solid, (0.100 g, 68 %); MS (ISP): 294.4 ((M+H)^{+.}).

### Example 60

### (3,4-Dichloro-phenyl)-(1H-imidazol-2-ylmethyl)-isopropyl-amine

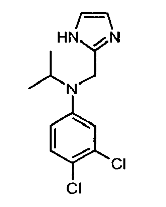

### a) (3,4-Dichloro-phenyl)-isopropyl-amine

3,4-Dichloroaniline (5.0 g, 30.86 mmol) was dissolved in methanol (150 ml). Then acetone (22.7 ml, 308.6 mmol), zinc chloride (12.62 g, 92.58 mmol) and sodium cyanoborohydride (7.76 g, 123.4 mmol) were added and the reaction mixture was allowed to stir at 40 °C overnight. After cooling, the reaction mixture was poured onto ammoniumchloride/ice and extracted with ethyl acetate (2 times 200 ml). The organic layer was dried over magnesium sulfate and evaporated. The residue was purified using flash chromatography (SiO₂; eluent: heptane/ ethyl acetate = 95:5) to yield an off-white solid (5.03 g, 79.9 %); MS (ISP): 205.1 ([³⁷Cl M+H]^{+.}), 203.1 ([³⁵Cl M+H]⁺).

### b) (3,4-Dichloro-phenyl)-(1H-imidazol-2-ylmethyl)-isopropyl-amine

(3,4-Dichloro-phenyl)-isopropyl-amine (0.30 g, 1.47 mmol) was dissolved in methanol (10 ml). Then imidazole-2-carboxaldehyde (0.22g, 2.20 mmol), zinc chloride (0.60 g, 4.4 mmol) and sodium cyanoborohydride (0.19 g, 2.9 mmol) were added and the reaction mixture was allowed to stir at 60 °C overnight. After cooling, the reaction mixture was concentrated *in vacuo* and the residue was purified using flash chromatography (SiO₂; eluent: dichloromethane/methanol gradient) to yield a white solid (0.007 g, 2 %); MS (ISP): 286.0 ([³⁷Cl M+H]^{+.}), 284.0 ([³⁵Cl M+H]⁺).

### Example 61

### (1H-Imidazol-2-ylmethyl)-(3-phenoxy-phenyl)-amine

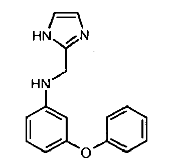

The title compound, MS (ISP): 266.2 ([M+H]⁺) was obtained in comparable yield analogous to the procedure described for Example 11 using 3-phenoxyaniline instead of 2-isopropyl-6-methyl-aniline.

### Example 62

### (3-Benzyloxy-phenyl)-(1H-imidazol-2-ylmethyl)-amine

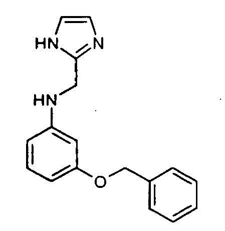

The title compound, MS (ISP): 280.2 ([M+H]⁺) was obtained in comparable yield analogous to the procedure described for Example 11 using 3-benzyloxyaniline instead of 2-isopropyl-6-methyl-aniline.

### Example 63

### (4-Benzyloxy-phenyl)-(1H-imidazol-2-ylmethyl)-amine

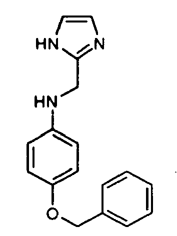

The title compound, MS (ISP): 280.0 ([M+H]⁺) was obtained in comparable yield analogous to the procedure described for Example 11 using 4-benzyloxyaniline instead of 2-isopropyl-6-methyl-aniline.

### Example 64

### (4-Chloro-3-methoxy-phenyl)-(1H-imidazol-2-ylmethyl)-amine

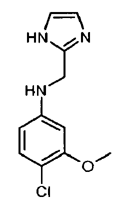

The title compound, MS (ISP): 240.2 ([³⁷Cl M+H]^{+.}), 238.0 ([³⁵Cl M+H]^{+.}) was obtained in comparable yield analogous to the procedure described for Example 11 using 4-chloro-3-methoxyaniline instead of 2-isopropyl-6-methyl-aniline.

### Example 65

### Ethyl-(1H-imidazol-2-ylmethyl)-(3-phenoxy-phenyl)-amine

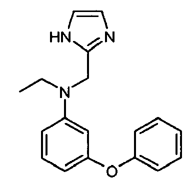

The title compound, MS (1SP): 294.2 ((M+H)^{+.}) was obtained in comparable yield analogous to the procedure described for Example 12 step b) using (1H-imidazol-2-ylmethyl)-(3-phenoxy-phenyl)-amine (Example 61) instead of (1H-imidazol-2-ylmethyl)-(3-fluoro-phenyl)-amine.

### Example 66

### (3-Benzyloxy-phenyl)-ethyl-(1H-imidazol-2-ylmethyl)-amine

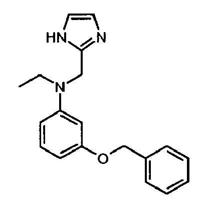

The title compound, MS (ISP): 308.4 ((M+H)^{+.}) was obtained in comparable yield analogous to the procedure described for Example 12 step b) using (3-benzyloxyphenyl)-(1H-imidazol-2-ylmethyl)-amine (Example 62) instead of (1H-imidazol-2-ylmethyl)-(3-fluoro-phenyl)-amine.

### Example 67

### (4-Benzyloxy-phenyl)-ethyl-(1H-imidazol-2-ylmethyl)-amine

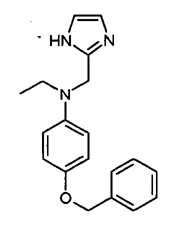

The title compound, MS (ISP): 308.3 ((M+H)^{+.}) was obtained in comparable yield analogous to the procedure described for Example 12 step b) using (4-benzyloxyphenyl)-(1H-imidazol-2-ylmethyl)-amine (Example 63) instead of (1H-imidazol-2-ylmethyl)-(3-fluoro-phenyl)-amine.

### Example 68

### (3,4-Dichloro-phenyl)-ethyl-(1H-imidazol-2-ylmethyl)-amine

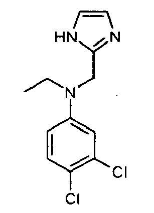

The title compound, MS (ISP): 272.1 ([³⁷Cl M+H]^{+.}), 270.2 ([³⁵ClM+H]^{+.}) was obtained in comparable yield analogous to the procedure described for Example 12 using 3,4-dichloroaniline in place of 3-fluoroaniline in step a) and using (3,4-dichloro-phenyl)-(1H-imidazo)-2-ylmethyl)-amine instead of (1H-imidazol-2-ylmethyl)-(3-fluorophenyl)-amine in step b.

### Example 69

### (4-Chloro-3-methoxy-phenyl)-(1H-imidazol-2-ylmethyl)-methyl-amine

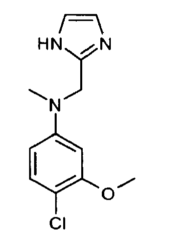

(4-Chloro-3-methoxy-phenyl)-(1H-imidazol-2-ylmethyl)-amine (0.10 g, 0.4 mmol) (Example 64) was dissolved in acetonitrile(4 ml). Then formaldehyde (0.08 ml, 1.1 mmol, 37% aq solution) and sodium cyanoborohydride (0.08 g, 1.3 mmol) were added and the reaction mixture was allowed to stir at room temperature overnight. The reaction mixture was concentrated *in vacuo* and the residue was purified using flash chromatography (SiO₂; eluent: methanol/dichloromethane gradient) to yield a white solid (0.006 g, 6 %); MS (ISP): 252.1 ([³⁷Cl M+H]^{+.}), 250.2 ([³⁵Cl M+H]^{+.}).

### Example 70

### (4-Chloro-3-methoxy-phenyl)-(1H-imidazol-2-ylmethyl)-isopropyl-amine

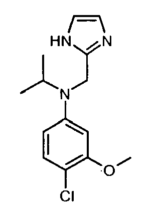

The title compound, MS (ISP): 282.1 ([³⁷ClM+H]^{+.}), 280.2 ([³⁵Cl M+H]^{+.}). was obtained in comparable yield analogous to the procedure described for Example 1 step b) using (4-chloro-3-methoxy-phenyl)-(1H-imidazol-2-ylmethyl)-amine (Example 64) instead of (1H-imidazol-2-ylmethyl)-phenyl-amine.

### Example 71

### (3-Bromo-phenyl)-(1H-imidazol-2-ylmethyl)-amine

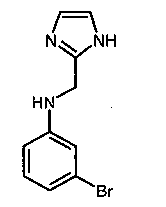

The title compound, MS (ISP): 251.9; 254.1 ((M+H)^{+.}) was obtained in comparable yield analogous to the procedure described for Example 11 using 3-bromoaniline instead of 2-isopropyl-6-methyl-aniline.

### Example 72

### (1H-Imidazol-2-ylmethyl)-(4-methoxy-phenyl)-amine

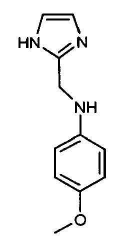

The title compound, MS (ISP): 203.9 ((M+H)^{+.}) was obtained in comparable yield analogous to the procedure described for Example 11 using 4-methoxyaniline instead of 2-isopropyl-6-methyl-aniline.

### Example 73

### (3,4-Difluoro-phenyl)-(1H-imidazol-2-ylmethyl)-amine

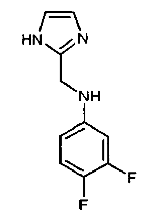

The title compound, MS (ISP): 209.9 ((M+H)^{+.}) was obtained in comparable yield analogous to the procedure described for Example 11 using 3,4-difluoroaniline instead of 2-isopropyl-6-methyl-aniline.

### Example 74

### (3-Chloro-4-fluoro-phenyl)-(1H-imidazol-2-ylmethyl)-amine

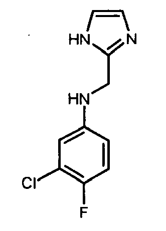

The title compound, MS (ISP): 228.1 ([³⁷Cl M+H]^{+.}), 226.1 ([³⁵Cl M+H]^{+.}) was obtained in comparable yield analogous to the procedure described for Example 11 using 3-chloro-4-fluoroaniline instead of 2-isopropyl-6-methyl-aniline.

### Example 75

### (3-Bromo-phenyl)-(1H-imidazol-2-ylmethyl)-isopropyl-amine

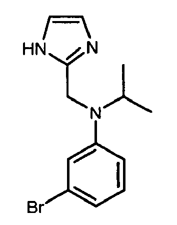

The title compound, MS (ISP): 296.1; 294.1 ((M+H)^{+.}) was obtained in comparable yield analogous to the procedure described for Example 1 using 3-bromoaniline instead of aniline in step a).

### Example 76

### (1H-Imidazol-2-ylmethyl)-isopropyl-(4-methoxy-phenyl)-amine

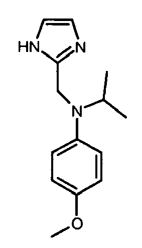

The title compound, MS (ISP): 246.2 ((M+H)^{+.}) was obtained in comparable yield analogous to the procedure described for Example 1 using 4-methoxyaniline instead of aniline in step a).

### Example 77

### (3,4-Difluoro-phenyl)-(1H-imidazol-2-ylmethyl)-isopropyl-amine

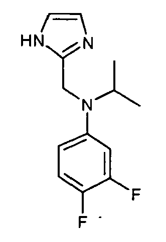

The title compound, MS (ISP): 252.1 ((M+H)^{+.}) was obtained in comparable yield analogous to the procedure described for Example 1 using 3,4-difluoroaniline instead of aniline in step a).

### Example 78

### (3-Chloro-4-fluoro-phenyl)-(1H-imidazol-2-ylmethyl)-isopropyl-amine

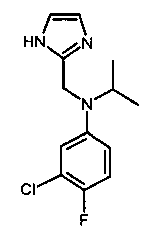

The title compound, MS (ISP): 268.1; 270.1 ((M+H)^{+.}) was obtained in comparable yield analogous to the procedure described for Example 1 using 3-chloro-4-fluoroaniline instead of aniline in step a).

### Example 79

### (3-Bromo-phenyl)-ethyl-(1H-imidazol-2-ylmethyl)-amine

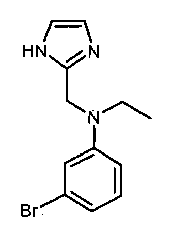

The title compound, MS (ISP): 279.9; 281.9 ((M+H)⁺) was obtained in comparable yield analogous to the procedure described for Example 1 using 3-bromoaniline instead of aniline in step a).

### Example 80

### Ethyl-(1H-imidazol-2-ylmethyl)-(4-methoxy-phenyl)-amine

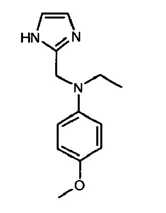

The title compound, MS (ISP): 231.9 ((M+H)⁺) was obtained in comparable yield analogous to the procedure described for Example 12 using using 4-methoxyaniline instead of 3-fluoroaniline in step a).

### Example 81

### (3,4-Difluoro-phenyl)-ethyl-(1H-imidazol-2-ylmethyl)-amine

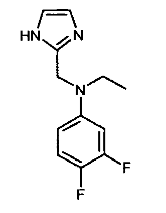

The title compound, MS (ISP): 237.9 ((M+H)⁺_{˙}) was obtained in comparable yield analogous to the procedure described for Example 12 using using 3,4-difluoroaniline instead of 3-fluoroaniline in step a).

### Example 82

### (3-Chloro-4-fluoro-phenyl)-ethyl-(1H-imidazol-2-ylmethyl)-amine

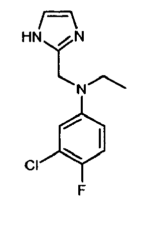

The title compound, MS (ISP): 253.9; 255.8 ((M+H)⁺·) was obtained in comparable yield analogous to the procedure described for Example 12 using using 3-chloro-4-fluoroaniline instead of 3-fluoroaniline in step a).

### Example 83

### 2-[Biphenyl-3-yl-(1H-imidazol-2-ylmethyl)-amino]-ethanol

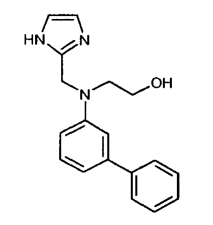

The title compound, MS (ISP): 294.1 ((M+H)^{+·}) was obtained in comparable yield analogous to the procedure described for Example 2 using 2-(biphenyl-3-ylamino)-ethanol instead of 3-chloro-N-methylaniline.

## Claims

1. Compounds of formula I 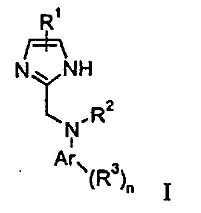 wherein
R¹ is hydrogen or C₁₋₇-alkyl;
R² is C₁₋₇-alkyl, C₂₋₇-alkenyl, C₁₋₇-alkyl substituted by hydroxy,
C₁₋₇-alkyl substituted by halogen,
-(CH₂)ₓ-S-C₁₋₇-alkyl,
-(CH₂)ₓ-O-C₁₋₇-alkyl,
-(CH₂)ₓ-NHC(O)O-C₁₋₇-alkyl,
-(CH₂)ₓ-aryl or
-(CH₂)ₓ-heteroaryl;
R³ is hydrogen, C₁₋₇-alkyl, C₁₋₇-alkoxy, halogen, hydroxy, C₁₋₇-alkyl
substituted by halogen,
-O-(CH₂)ₘ-aryl,
-O-(CH₂)ₘ-heteroaryl,
-(CR₂)ₘ-aryl or
-(CR₂)ₘ-heteroaryl;
R is hydrogen, C₁₋₇-alkyl or hydroxy;
Ar is phenyl, pyrimidin-2-yl, pyrimidin-4-yl or pyridin-3-yl;
n is 0, I or 2; when n is 2, then the two R³ groups may or may not be the same;
x is 0,1,2 or 3;
m is 0 or 1; when m is 1, then the two R groups may or may not be the same;
and to their pharmaceutically active salts, with the exception of the following known compounds:
benzyl-(*1H*-imidazol-2-ylmethyl)-phenyl-amine and
ethyl-(*1H*-imidazol-2-ylmethyl)-phenyl-amine.

2. Compounds of formula 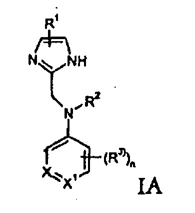 wherein
R¹ is hydrogen or C₁₋₇-alkyl;
R² is C₁₋₇-alkyl, C₂₋₇-alkenyl, C₁₋₇-alkyl substituted by hydroxy,
C₁₋₇-alkyl substituted by halogen,
-(CH₂)ₓ-S-C₁₋₇-alkyl,
-(CH₂)ₓ-O-C₁₋₇-alkyl,
-(CH₂)ₓ-NHC(O)O- C₁₋₇-alkyl,
-(CH₂)ₓ-aryl or
-(CH₂)ₓ-heteroaryl;
R³ is hydrogen, C₁₋₇-alkyl, C₁₋₇-alkoxy, halogen, hydroxy, C₁₋₇-alkyl substituted by halogen,
-O-(CH₂)ₘ-aryl,
-O-(CH₂)ₘ-heteroaryl,
-(CR₂)ₘ-aryl or
-(CR₂)ₘ-heteroaryl;
R is hydrogen, C₁₋₇-alkyl or hydroxy;
X/X¹ are independently CH or N, wherein X and X¹ are not simultaneously N;
n is 0,1 or 2; when n is 2, then the two R³ groups may or may not be the same;
x is 0,1,2 or 3;
m is 0 or 1; when m is 1, then the two R groups may or may not be the same;
and their pharmaceutically active salts, with the exception of the following known compounds:
benzyl-(*1H*-imidazol-2-ylmethyl)-phenyl-amine and
ethyl-(*1H*-imidazol-2-ylmethyl)-phenyl-amine.

3. Compounds of formula I wherein Ar is phenyl.

4. Compounds of formula I according to claim 3, wherein R² is C₁₋₇-alkyl.

5. Compounds of formula I according to claim 4, which compounds are
(3-chloro-phenyl)-(1H-imidazol-2-ylmethyl)-methyl-amine
(4-chloro-phenyl)-(1H-imidazol-2-ylmethyl)-methyl-amine
(1H-imidazol-2-ylmethyl)-(3-methoxy-phenyl)-methyl-amine
(4-fluoro-phenyl)-(1H-imidazol-2-ylmethyl)-isopropyl-amine
ethyl-(3-fluoro-phenyl)-(1H-imidazol-2-ylmethyl)-amine
(3-chloro-phenyl)-(1H-imidazol-2-ylmethyl)-isopropyl-amine
(3-chloro-phenyl)-ethyl-(1H-imidazol-2-ylmethyl)-amine
(2,5-difluoro-phenyl)-(3H-imidazol-4-ylmethyl)-isopropyl-amine
(1H-imidazol-2-ylmethyl)-isopropyl-m-tolyl-amine
(3-benzyloxy-phenyl)-(1H-imidazol-2-ylmethyl)-isopropyl-amine
(1H-imidazol-2-ylmethyl)-isopropyl-[3-(pyridin-3-yloxy)-phenyl]-amine
(3-benzyl-phenyl)-(1H-imidazol-2-ylmethyl)-isopropyl-amine
biphenyl-3-yl-(1H-imidazol-2-ylmethyl)-isopropyl-amine
[3-(4-chloro-phenoxy)-phenyl]-(1H-imidazol-2-ylmethyl)-isopropyl-amine
Ethyl-(1H-imidazol-2-ylmethyl)-(3-phenoxy-phenyl)-amine
(3-benzyloxy-phenyl)-ethyl-(1H-imidazol-2-ylmethyl)-amine or
(3,4-dichloro-phenyl)-ethyl-(1H-imidazol-2-ylmethyl)-amine.

6. Compounds of formula I according to claim 3, wherein R² is
-(CH₂)ₓ-O-C₁₋₇-alkyl.

7. Compounds of formula I according to claim 6, which compound is
(1H-imidazol-2-ylmethyl)-(2-methoxy-ethyl)-phenyl-amine.

8. Compounds of formula I according to claim 1 wherein Ar is pyrimidin-2-yl.

9. Compounds of formula I according to claim 1 wherein Ar is pyrimidin-4-yl.

10. Compounds of formula I according to claim 1 wherein Ar is pyridin-3-yl.

11. A process for preparation of compounds of formula I, which process comprises
a) reacting a compound of formula 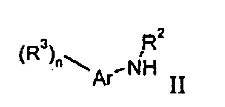 with a compound of formula 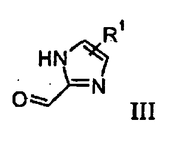 to a compound of formula 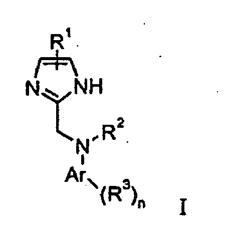 wherein R¹, R², R³, n and Ar are as defined above, or
b) reacting a compound of formula 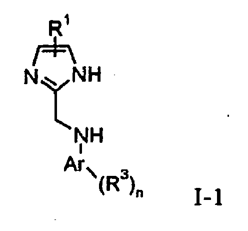 with a compound of formula R^{2'}-CHO
to a compound of formula 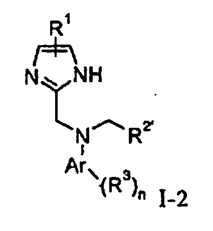 wherein R^{2'} is lower alkyl, lower alkenyl, lower alkyl substituted by hydroxy, lower alkyl substituted by halogen, -(CH₂)ₓ₋₁-S-lower alkyl, (CH₂)ₓ₋₁-O-lower alkyl, (CH₂)ₓ₋₁-NHC(O)O-lower alkyl or (CH₂)ₓ₋₁-heteroaryl and the other substituents are as defined above, or
c) reacting a compound of formula 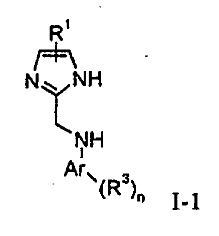 with 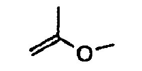 to a compound of formula 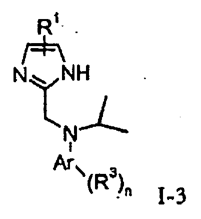 wherein the substituents are as defined above, or
d) removing a protecting group of compounds of formulas 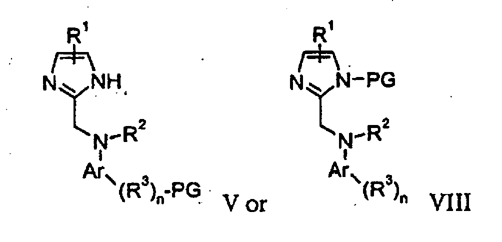 to a compound of formula 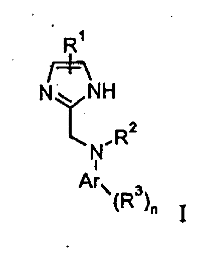 wherein the substituents are as defined above, or
e) reducing a compound of formula 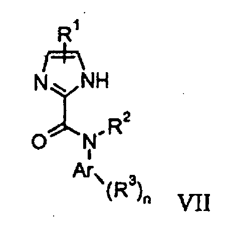 to a compound of formula 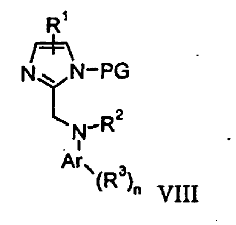 and removing the protecting group to a compound of formula I, wherein the substituents are as defined above, or
f) reacting a compound of formula 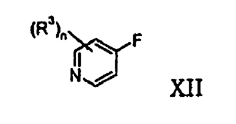 with a compound of formula 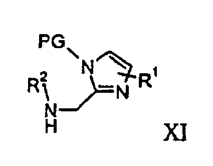 to a compound of formula 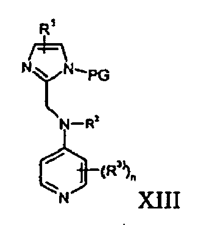 and removing the protecting group to a compound of formula 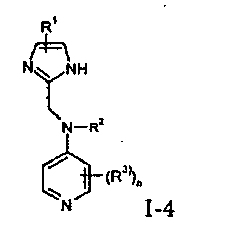
wherein the substituents are as defined above, and
if desired, converting the compounds obtained into pharmaceutically acceptable acid addition salts.

12. A compound according to claim 1, whenever prepared by a process as claimed in claim 11 or by an equivalent method.

13. A medicament containing one or more compounds of formula I and of compounds
benzyl-(*1H*-imidazol-2-ylmethyl)-phenyl-amine and
ethyl-(*1H*-imidaol-2-ylmethyl)-phenyl-amine
in accordance with claim 1 and pharmaceutically acceptable excipients.

14. A medicament as claimed in claim 13 for the treatment of depression, anxiety disorders, bipolar disorder, attention deficit hyperactivity disorder, stress-related disorders, psychotic disorders, schizophrenia, neurological diseases, Parkinson's disease, neurodegenerative disorders, Alzheimer's disease, epilepsy, migraine, hypertension, substance abuse and metabolic disorders, eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

15. A medicament according to claim 14 containing one or more compounds as claimed in claim 1 for the treatment of depression, psychosis, Parkinson's disease, anxiety and attention deficit hyperactivity disorder (ADHD).

16. The use of a compound of formula I and of compounds
benzyl-(*1H*-imidazol-2-ylmethyl)-phenyl-amine and
ethyl- (*1H*-imidazol-2-ylmethyl)-phenyl-amine
according to claim 1 for the preparation of a medicament for the treatment of depression, anxiety disorders, bipolar disorder, attention deficit hyperactivity disorder, stress-related disorders, psychotic disorders, schizophrenia, neurological diseases, Parkinson's disease, neurodegenerative disorders, Alzheimer's disease, epilepsy, migraine, hypertension, substance abuse and metabolic disorders, eating disorders, diabetes, diabetic complications, obesity, dyslipidemia, disorders of energy consumption and assimilation, disorders and malfunction of body temperature homeostasis, disorders of sleep and circadian rhythm, and cardiovascular disorders.

## Patentansprüche

1. Verbindungen der Formel I 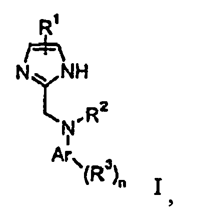 worin
R¹ Wasserstoff oder C₁₋₇Alkyl ist;
R² C₁₋₇-Alkyl, C₂₋₇-Alkenyl, C₁₋₇-Alkyl, substituiert durch Hydroxy,
C₁₋₇-Alkyl, substituiert durch Halogen,
-(CH₂)ₓ-S-C₁₋₇-Alkyl,
-(CH₂)ₓ-O-C₁₋₇-Alkyl,
-(CH₂)ₓ-NHC(O)O-C₁₋₇-Alkyl,
-(CH₂)ₓ-Aryl oder
-(CH₂)ₓ-Heteroaryl ist;
R³ Wasserstoff, C₁₋₇-Alkyl, C₁₋₇-Alkoxy, Halogen, Hydroxy, C₁₋₇-Alkyl, substituiert durch Halogen,
-O-(CH₂)ₘ-Aryl,
-O-(CH₂)ₘ-Heteroaryl,
-(CR₂)ₘ-Aryl oder
-(CR₂)ₘ-Heteroaryl ist;
R Wasserstoff, C₁₋₇-Alkyl oder Hydroxy ist;
Ar Phenyl, Pyrimidin-2-yl, Pyrimidin-4-yl oder Pyridin-3-yl ist;
n 0, 1 oder 2 ist; wenn n 2 ist, dann die zwei R³-Gruppen dieselben sein können oder nicht;
x 0,1,2 oder 3 ist;
m 0 oder 1 ist; wenn m 1 ist, dann die zwei R-Gruppen dieselben sein können oder nicht;
und deren pharmazeutisch aktive Salze mit Ausnahme der folgenden bekannten Verbindungen:
Benzyl-(*1H*-imidazol-2-ylmethyl)-phenyl-amin und
Ethyl-(*1H*-imidazol-2-ylmethyl)-phenyl-amin.

2. Verbindungen der Formel 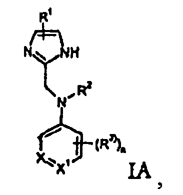 worin
R¹ Wasserstoff oder C₁₋₇-Alkyl ist;
R² C₁₋₇-Alkyl, C₂₋₇-Alkenyl, C₁₋₇-Alkyl, substituiert durch Hydroxy,
C₁₋₇-Alkyl, substituiert durch Halogen,
-(CH₂)ₓ-S-C₁₋₇-Alkyl,
-(CH₂)ₓ-O-C₁₋₇-Alkyl,
-(CH₂)ₓ-NHC(O)O-C₁₋₇-Alkyl,
-(CH₂)ₓ-Aryl oder
-(CH₂)ₓ-Heteroaryl ist;
R³ Wasserstoff, C₁₋₇-Alkyl, C₁₋₇-Alkoxy, Halogen, Hydroxy, C₁₋₇-Alkyl, substituiert durch Halogen,
-O-(CH₂)ₘ-Aryl,
-O-(CH₂)ₘ-Heteroaryl;
-(CR₂)ₘ-Aryl oder
-(CR₂)ₘ-Heteroaryl ist;
R Wasserstoff, C₁₋₇-Alkyl oder Hydroxy ist;
X/X¹ unabhängig voneinander CH oder N sind, wobei X und X¹ nicht gleichzeitig N sind;
n 0, 1 oder 2 ist; wenn n 2 ist, dann die zwei R³-Gruppen dieselben sein können oder nicht;
x 0,1,2 oder 3 ist;
m 0 oder 1 ist; wenn m 1 ist, dann die zwei R-Gruppen dieselben sein können oder nicht;
und deren pharmazeutisch aktive Salze mit Ausnahme der folgenden bekannten Verbindungen:
Benzyl-(*1H*-imidazol-2-ylmethyl)-phenyl-amin und
Ethyl-(*1H*-imidazol-2-ylmethyl)-phenyl-amin.

3. Verbindungen der Formel I, wobei Ar Phenyl ist.

4. Verbindungen der Formel I nach Anspruch 3, wobei R² C₁₋₇-Alkyl ist.

5. Verbindungen der Formel I nach Anspruch 4, wobei die Verbindungen
(3-Chlor-phenyl)-(1H-imidazol-2-ylmethyl)-methyl-amin,
(4-Chlor-phenyl)-(1H-imidazol-2-ylmethyl)-methyl-amin,
(1H-Imidazol-2-ylmethyl)-(3-methoxy-phenyl)-methyl-amin,
(4-Fluor-phenyl)-(1H-imidazol-2-ylmethyl)-isopropyl-amin,
Ethyl-(3-fluor-phenyl)-(1H-imidazol-2-ylmethyl)-amin,
(3-Chlor-phenyl)-(1H-imidazol-2-ylmethyl)-isopropyl-amin,
(3-Chlor-phenyl)-ethyl-(1H-imidazol-2-ylmethyl)-amin,
(2,5-Difluor-phenyl)-(3H-imidazol-4-ylmethyl)-isopropyl-amin,
(1H-Imidazol-2-ylmethyl)-isopropyl-m-tolyl-amin,
(3-Benzyloxy-phenyl)-(1H-imidazol-2-ylmethyl)-isopropyl-amin,
(1H-Imidazol-2-ylmethyl)-isopropyl-[3-(pyridin-3-yloxy)-phenyl]-amin,
(3-Benzyl-phenyl)-(1H-imidazol-2-ylmethyl)-isopropyl-amin,
Biphenyl-3-yl-(1H-imidazol-2-ylmethyl)-isopropyl-amin,
[3-(4-Chlor-phenoxy)-phenyl]-(1H-imidazol-2-ylmethyl)-isopropyl-amin,
Ethyl-(1H-imidazol-2-ylmethyl)-(3-phenoxy-phenyl)-amin,
(3-Benzyloxy-phenyl)-ethyl-(1H-imidazol-2-ylmethyl)-amin oder
(3,4-Dichlor-phenyl)-ethyl-(1H-imidazol-2-ylmethyl)-amin sind.

6. Verbindungen der Formel I nach Anspruch 3, wobei R² -(CH₂)ₓ-O-C₁₋₇-Alkyl ist.

7. Verbindungen der Formel I nach Anspruch 6, wobei die Verbindung (1H-Imidazol-2-ylmethyl)-(2-methoxy-ethyl)-phenyl-amin ist.

8. Verbindungen der Formel I nach Anspruch 1, wobei Ar Pyrimidin-2-yl ist.

9. Verbindungen der Formel I nach Anspruch 1, wobei Ar Pyrimidin-4-yl ist.

10. Verbindungen der Formel I nach Anspruch 1, wobei Ar Pyridin-3-yl ist.

11. Verfahren zur Herstellung von Verbindungen der Formel I, wobei das Verfahren
a) das Umsetzen einer Verbindung der Formel 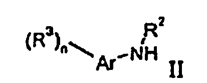 mit einer Verbindung der Formel 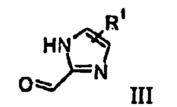 zu einer Verbindung der Formel 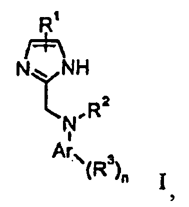 wobei R¹, R², R³, n und Ar wie oben definiert sind, oder
b) das Umsetzen einer Verbindung der Formel 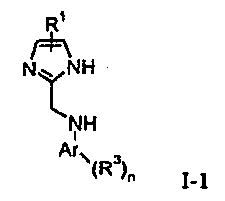 mit einer Verbindung der Formel R^{2'}-CHO
zu einer Verbindung der Formel 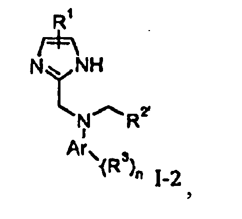 wobei R^{2'} Niederalkyl, Niederalkenyl, Niederalkyl, substituiert durch Hydroxy, Niederalkyl, substituiert durch Halogen, -(CH₂)ₓ₋₁-S-Niederalkyl, -(CH₂)ₓ₋₁-O-Niederalkyl, (CH₂)ₓ₋₁-NHC(O)O-Niederalkyl oder (CH₂)ₓ₋₁-Heteroaryl ist und die anderen Substituenten wie oben definiert sind, oder
c) das Umsetzen einer Verbindung der Formel 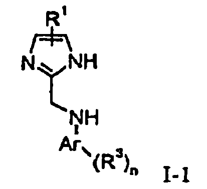 mit 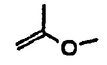 zu einer Verbindung der Formel 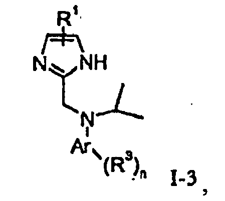 wobei die Substituenten wie oben definiert sind, oder
d) das Entfernen einer Schutzgruppe von Verbindungen der Formeln 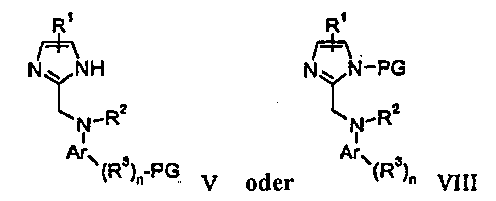 unter Erhalt einer Verbindung der Formel 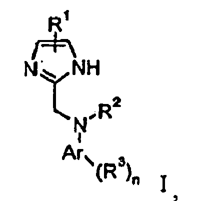 wobei die Substituenten wie oben definiert sind, oder
e) das Reduzieren einer Verbindung der Formel 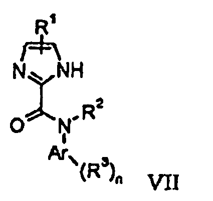 zu einer Verbindung der Formel 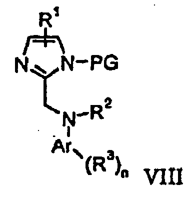 und das Entfernen der Schutzgruppe unter Erhalt einer Verbindung der Formel I, wobei die Substituenten wie oben definiert sind, oder
f) das Umsetzen einer Verbindung der Formel 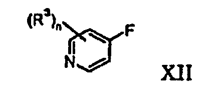 mit einer Verbindung der Formel 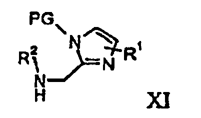 zu einer Verbindung der Formel 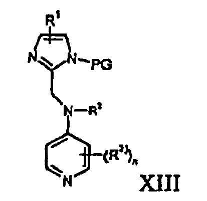 und das Entfernen der Schutzgruppe unter Erhalt einer Verbindung der Formel 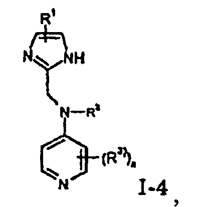
wobei die Substituenten wie oben definiert sind, und, wenn gewünscht, das Umwandeln der erhaltenen Verbindungen in pharmazeutisch akzeptable Säureadditionssalze umfasst.

12. Verbindung nach Anspruch 1, hergestellt durch ein Verfahren nach Anspruch 11 oder ein äquivalentes Verfahren.

13. Medikament, enthaltend eine oder mehrere Verbindungen der Formel I und die Verbindungen
Benzyl-(*1H*-imidazol-2-ylmethyl)-phenyl-amin und
Ethyl-(*1H*-imidazol-2-ylmethyl)-phenyl-amin
nach Anspruch 1 und pharmazeutisch akzeptable Hilfsstoffe.

14. Medikament nach Anspruch 13 zur Behandlung von Depression, Angststörungen, bipolarer Störung, Aufmerksamkeits- und Hyperaktivitätsstörung, Stress-bedingten Störungen, psychotischen Störungen, Schizophrenie, neurologischen Erkrankungen, Parkinson-Krankheit, neurodegenerativen Störungen, Alzheimer-Krankheit, Epilepsie, Migräne, Hypertension, Substanzmissbrauch und Stoffwechselstörungen, Essstörungen, Diabetes, diabetischen Komplikationen, Fettleibigkeit, Dyslipidämie, Störungen von Energieverbrauch und -assimilation, Störungen und Fehlfunktion der Körpertemperaturhomöostase, Schlaf- und Herzrhythmusstörungen und kardiovaskulären Störungen.

15. Medikament nach Anspruch 14, enthaltend eine oder mehrere Verbindungen nach Anspruch 1 zur Behandlung von Depression, Psychose, Parkinson-Krankheit, Angst und Aufmerksamkeits- und Hyperaktivitätsstörung (ADHD).

16. Verwendung einer Verbindung der Formel I und der Verbindungen Benzyl-(*1H*-imidazol-2-ylmethyl)-phenyl-amin und
Ethyl-(*1H*-imidazol-2-ylmethyl)-phenyl-amin
nach Anspruch 1 zur Herstellung eines Medikaments zur Behandlung von Depression, Angststörungen, bipolarer Störung, Aufmerksamkeits- und Hyperaktivitätsstörung, Stress-bedingten Störungen, psychotischen Störungen, Schizophrenie, neurologischen Erkrankungen, Parkinson-Krankheit, neurodegenerativen Störungen, Alzheimer-Krankheit, Epilepsie, Migräne, Hypertension, Substanzmissbrauch und Stoffwechselstörungen,Essstörungen, Diabetes, diabetischen Komplikationen, Fettleibigkeit, Dyslipidämie, Störungen von Energieverbrauch und -assimilation, Störungen und Fehlfunktion der Körpertemperaturhomöostase, Schlaf- und Herzrhythmusstörungen und kardiovaskulären Störungen.

## Revendications

1. Composés de formule I 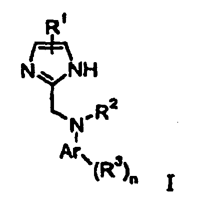 dans laquelle
R¹ est hydrogène ou alkyle en C₁-C₇;
R² est alkyle en C₁-C₇, alcényle en C₂-C₇, alkyle en C₁-C₇ substitué par hydroxy, alkyle en C₁-C₇ substitué par halogène,
-(CH₂)ₓ-S-(alkyle en C₁-C₇),
-(CH₂)ₓ-O-(alkyle en C₁-C₇),
-(CH₂)ₓ-NHC(O)O-(alkyle en C₁-C₇),
-(CH₂)ₓ-aryle ou
-(CH₂)ₓ-hétéroaryle;
R³ est hydrogène, alkyle en C₁-C₇, alcoxy en C₁-C₇, halogène, hydroxy, alkyle en C₁-C₇ substitué par halogène,
-O-(CH₂)ₘ-aryle,
-O-(CH₂)ₘ-hétéroaryle,
-C(R₂)ₘ-aryle ou
-C(R₂)ₘ-hétéroaryle;
R est hydrogène, alkyle en C₁-C₇ ou hydroxy;
Ar est phényle, pyrimidin-2-yle, pyrimidin-4-yle ou pyridin-3-yle;
n est 0, 1 ou 2; lorsque n est 2, les deux groupes R³ peuvent être identiques ou non;
x est 0,1,2 ou 3;
m est 0 ou 1; lorsque m est 1, les deux groupes R peuvent être identiques ou non;
et leurs sels pharmaceutiquement actifs, à l'exception des composés connus suivants:
la benzyl-(1H-imidazol-2-ylméthyl)-phénylamine et
l'éthyl-(1H-imidazol-2-ylméthyl)-phénylamine.

2. Composés de formule 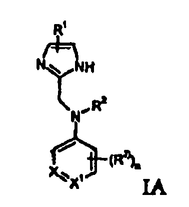 dans laquelle
R¹ est hydrogène ou alkyle en C₁-C₇;
R² est alkyle en C₁-C₇, alcényle en C₂-C₇, alkyle en C₁-C₇ substitué par hydroxy, alkyle en C₁-C₇ substitué par halogène,
-(CH₂)ₓ-S-(alkyle en C₁-C₇),
-(CH₂)ₓ-O-(alkyle en C₁-C₇),
-(CH₂)ₓ-NHC(O)O-(alkyle en C₁-C₇),
-(CH₂)ₓ-aryle ou
-(CH₂)ₓ-hétéroaryle;
R³ est hydrogène, alkyle en C₁-C₇, alcoxy en C₁-C₇, halogène, hydroxy, alkyle en
C₁-C₇ substitué par halogène,
-O-(CH₂)ₘ-aryle,
-O-(CH₂)ₘ-hétéroaryle,
-C(R₂)ₘ-aryle ou
-C(R₂)ₘ-hétéroaryle;
R est hydrogène, alkyle en C₁-C₇ ou hydroxy;
X/X¹ sont indépendamment CH ou N, X et X¹ n'étant pas N simultanément;
n est 0, 1 ou 2; lorsque n est 2, les deux groupes R³ peuvent être identiques ou non;
x est 0,1,2 ou 3;
m est 0 ou 1; lorsque m est 1, les deux groupes R peuvent être identiques ou non;
et leurs sels pharmaceutiquement actifs, à l'exception des composés connus suivants:
la benzyl-(1H-imidazol-2-ylméthyl)-phénylamine et
l'éthyl-(1H-imidazol-2-ylméthyl)-phénylamine.

3. Composés de formule I, dans lesquels Ar est phényle.

4. Composés de formule I selon la revendication 3, dans lesquels R² est alkyle en C₁-C₇.

5. Composés de formule I selon la revendication 4, ces composés étant:
la (3-chlorophényl)-(1H-imidazol-2-ylméthyl)-méthylamine,
la (4-chlorophényl)-(1H-imidazol-2-ylméthyl)-méthylamine,
la (1H-imidazol-2-ylméthyl)-(3-méthoxyphényl)-méthylamine,
la (4-fluorophényl)-(1H-imidazol-2-ylméthyl)-isopropylamine,
l'éthyl-(3 -fluorophényl)-(1H-imidazol-2-ylméthyl)amine,
la (3-chlorophényl)-(1H-imidazol-2-ylméthyl)-isopropylamine,
la (3-chlorophényl)-éthyl-(1H-imidazol-2-ylméthyl)amine,
la (2,5-difluorophényl)-(3H-imidazol-4-ylméthyl)-isopropylamine,
la (1H-imidazol-2-ylméthyl)-isopropyl-m-tolylamine,
la (3-benzyloxyphényl)-(1H-imidazol-2-ylméthyl)-isopropylamine,
la (1H-imidazol-2-ylméthyl)-isopropyl-[3-(pyridin-3-yloxy)phényl]amine,
la (3-benzylphényl)-(1H-imidazol-2-ylméthyl)-isopropylamine,
la biphényl-3-yl-(1H-imidazol-2-ylméthyl)-isopropylamine,
la [3-(4-chlorophénoxy)phényl]-(1H-imidazol-2-ylméthyl)-isopropylamine,
l'éthyl-(1H-imidazol-2-ylméthyl)-(3-phénoxyphényl)amine,
la (3-benzyloxyphényl)-éthyl-(1H-imidazol-2-ylméthyl)amine, ou
la (3,4-dichlorophényl)-éthyl-(1H-imidazol-2-ylméthyl)amine.

6. Composés de formule I selon la revendication 3, dans lesquels R² est -(CH₂)ₓ-O-(alkyle en C₁-C₇).

7. Composé de formule I selon la revendication 6, ce composé étant: la (1H-imidazol-2-ylméthyl)-(2-méthoxyéthyl)-phénylamine.

8. Composés de formule I selon la revendication 1, dans lesquels Ar est pyrimidin-2-yle.

9. Composés de formule I selon la revendication 1, dans lesquels Ar est pyrimidin-4-yle.

10. Composés de formule I selon la revendication 1, dans lesquels Ar est pyridin-3-yle.

11. Procédé de préparation de composés de formule I, ce procédé comprenant
a) la réaction d'un composé de formule 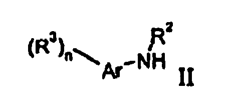 avec un composé de formule 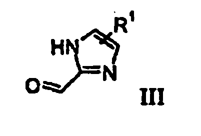 donnant un composé de formule 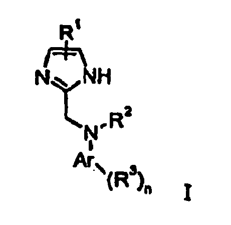 où R¹, R², R³, n et Ar sont tels que définis ci-dessus, ou
b) la réaction d'un composé de formule 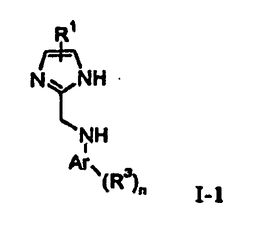 avec un composé de formule R^{2'}-CHO
donnant un composé de formule 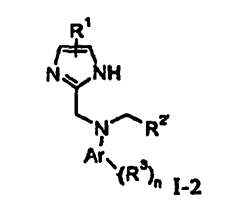 où R^{2'} est alkyle inférieur, alcényle inférieur, alkyle inférieur substitué par hydroxy, alkyle inférieur substitué par halogène, -(CH₂)ₓ₋₁-S-alkyle inférieur, -(CH₂)_{X-1}-O-alkyle inférieur, -(CH₂)ₓ₋₁-NHC(O)O-alkyle inférieur ou -(CH₂)ₓ₋₁-hétéroaryle, et les autres substituants sont tels que définis ci-dessus, ou
c) la réaction d'un composé de formule 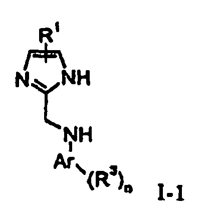 avec 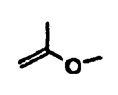 donnant un composé de formule 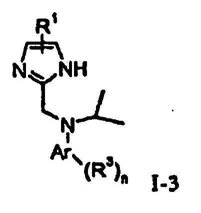 où les substituants sont tels que définis ci-dessus, ou
d) la séparation d'un groupe protecteur de composés de formule 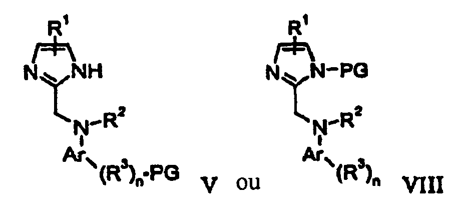 donnant un composé de formule 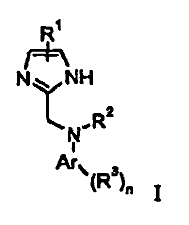 où les substituants sont tels que définis ci-dessus, ou
e) la réduction d'un composé de formule 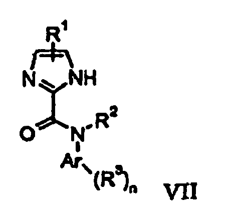 donnant un composé de formule 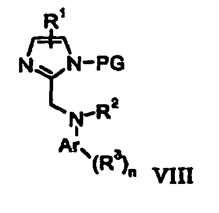 et la séparation du groupe protecteur pour l'obtention d'un composé de formule I, où tous les substituants sont tels que définis ci-dessus, ou
f) la réaction d'un composé de formule 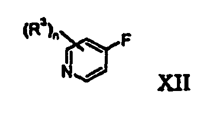 avec un composé de formule 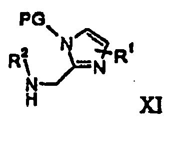 donnant un composé de formule 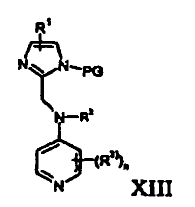 et la séparation du groupe protecteur pour l'obtention d'un composé de formule 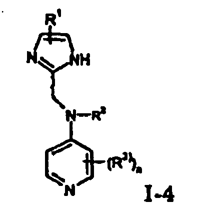 où les substituants sont tels que définis ci-dessus, et
si désiré, la conversion des composés obtenus en sels d'addition d'acides pharmaceutiquement acceptables.

12. Composé selon la revendication 1, préparé par un procédé selon la revendication 11 ou par un procédé équivalent.

13. Médicament contenant un ou plusieurs des composés de formule I et des composés
benzyl-(1H-imidazol-2-ylméthyl)-phénylamine et
éthyl-(1H-imidazol-2-ylméthyl)-phénylamine
selon la revendication 1 et des excipients pharmaceutiquement acceptables.

14. Médicament selon la revendication 13 pour le traitement de la dépression, de troubles de l'anxiété, de troubles bipolaires, de troubles d'hyperactivité avec déficit de l'attention, de troubles associés au stress, de troubles psychotiques, de la schizophrénie, de maladies neurologiques, de la maladie de Parkinson, de troubles neurodégénératifs, de la maladie d'Alzheimer, de l'épilepsie, de la migraine, de l'hypertension, de la toxicomanie et de troubles métaboliques, de troubles de l'alimentation, du diabète, des complications diabétiques, de l'obésité, de la dyslipidémie, de troubles de la consommation et de l'assimilation d'énergie, de troubles et du mauvais fonctionnement de l'homéostasie de la température corporelle, de troubles du sommeil et du rythme circadien, et de troubles cardio-vasculaires.

15. Médicament selon la revendication 14, contenant un ou plusieurs composés selon la revendication 1 pour le traitement de la dépression, de la psychose, de la maladie de Parkinson, de l'anxiété et des troubles d'hyperactivité avec déficit de l'attention (THDA).

16. Utilisation d'un composé de formule I et des composés
benzyl-(1H-imidazol-2-ylméthyl)-phénylamine et
éthyl-(1H-imidazol-2-ylméthyl)-phénylamine
selon la revendication 1 pour la préparation d'un médicament destiné au traitement de la dépression, de troubles de l'anxiété, de troubles bipolaires, de troubles d'hyperactivité avec déficit de l'attention, de troubles associés au stress, de troubles psychotiques, de la schizophrénie, de maladies neurologiques, de la maladie de Parkinson, de troubles neurodégénératifs, de la maladie d'Alzheimer, de l'épilepsie, de la migraine, de l'hypertension, de la toxicomanie et de troubles métaboliques, de troubles de l'alimentation, du diabète, des complications diabétiques, de l'obésité, de la dyslipidémie, de troubles de la consommation et de l'assimilation d'énergie, de troubles et du mauvais fonctionnement de l'homéostasie de la température corporelle, de troubles du sommeil et du rythme circadien, et de troubles cardio-vasculaires.
